# EUROPEAN PATENT SPECIFICATION

(11) **EP 4 577 889 B1**
(45) Date of publication and mention of the grant of the patent: **22.07.2026**
(21) Application number: 23769021.9
(22) Date of filing: 22.08.2023
(51) Int. Cl.: G06F 1/16, G04G 21/02, A61B 5/00, A61B 5/024, G06F 1/3234, G06F 1/3231, A61B 5/01, A61B 5/0205, A61B 5/0533, A61B 5/256, G04G 9/00, G04G 21/00, G04G 21/04, G06F 3/01

(54) **SYSTEM AND METHOD FOR DETERMINING A WEAR STATE OF A WEARABLE COMPUTING DEVICE**
SYSTEM UND VERFAHREN ZUR BESTIMMUNG EINES VERSCHLEISSZUSTANDS EINER TRAGBAREN RECHNERVORRICHTUNG
SYSTÈME ET PROCÉDÉ POUR DÉTERMINER UN ÉTAT D'USURE D'UN DISPOSITIF INFORMATIQUE VESTIMENTAIRE

(30) Priority: 23.08.2022 US 202263400339 P
(43) Date of publication of application: 02.07.2025
(73) Proprietor: Google LLC, Mountain View, CA 94043 (US)
(72) Inventor: GUTSCHICK, David Duncanson, Mountain View, California 94043 (US); WASSON, II, Zachariah Lord, Mountain View, California 94043 (US); ABDEL-GHAFFAR, Samy Ahmed Mansour, Mountain View, California 94043 (US); KERNASOVSKIY, Sarah Ann Stokes, Mountain View, California 94043 (US); MAIWAND, Heiko, Mountain View, California 94043 (US); GHENEA, Catalin, 011861 Bucuresti (RO); MAFTEI, Stefan Radu, 011861 Bucuresti (RO); KALINOWSKI, David Alexander, Mountain View, California 94043 (US); PARK, Junyong, Mountain View, California 94043 (US); WOODWARD, Steven Thomas, Mountain View, California 94043 (US); RIOT, Benjamin Patrick, Mountain View, California 94043 (US); SUNDEN, Lindsey Michelle, Mountain View, California 94043 (US); THOMSON, Seamus David, Mountain View, California 94043 (US); BORRA, Jeffrey Michael, Mountain View, California 94043 (US); NGUYEN, Thi Cac, Mountain View, California 94043 (US); MASTRANGELO, Luke David, Mountain View, California 94043 (US); KANE, Matthew Joseph, Mountain View, California 94043 (US)
(74) Representative: Maikowski & Ninnemann Patentanwälte Partnerschaft mbB
(86) International application number: PCT/US2023/030813
(87) International publication number: WO 2024/044180

(56) References cited:
- EP-A2- 3 811 854
- CN-A- 107 894 840
- US-A1- 2015 135 310

## Description

### FIELD OF THE INVENTION

The present disclosure relates generally to wearable computing devices, and more particularly, to systems and methods for determining a wear state of a wearable computing device.

### BACKGROUND

Recent advances in technology, including those available through consumer devices, have provided for corresponding advances in health detection and monitoring. For example, wearable computing devices such as fitness trackers and smart watches are able to determine information relating to the pulse or motion of a person wearing the device. For instance, certain biometric monitoring devices include a variety of sensors for measuring multiple biological parameters that can be beneficial to a user of the device, such as a heart rate sensor, multi-purpose electrical sensors compatible with electrocardiogram (ECG) and electrodermal activity (EDA) applications, infrared sensors, a gyroscope, an altimeter, an accelerometer, a temperature sensor, an ambient light sensor, Wi-Fi, GPS, a vibration sensor, a speaker, and a microphone, among others. Due to capabilities of conventional devices, it may be difficult to determine when the devices are being worn by a user and when the devices are properly fitted to the user, which can affect the accuracy of biometric measurements. Methods for determining a wear state of a wearable computing device are disclosed in EP 3 811 854 A2, US 2015/135310 A1 and CN 107 894 840 A.

In particular, prior art EP 3 811 854 A2 discloses a method for determining a wear state of a wearable computing device comprising: obtaining proximity data from an optical proximity sensor of the wearable computing device and impedance data from an impedance sensor of the wearable computing device, and determining that the wear state of the wearable computing device corresponds to one of an off-wrist state, and two wearing states of different quality based on the impedance data and the proximity data.

Accordingly, improved systems and methods for monitoring a wear state of a wearable computing device would be welcomed in the art.

### SUMMARY OF THE INVENTION

Aspects and advantages of embodiments of the present disclosure will be set forth in part in the following description, or can be learned from the description, or can be learned through practice of the embodiments. A claimed solution is specified by a computer-implemented method for determining a wear state of a wearable computing device according to claim 1 and by a wearable computing device according to claim 12. Dependent claims specify embodiments thereof.

In one aspect, a computer-implemented method for determining a wear state of a wearable computing device is provided. The method may include obtaining, by one or more processors of the wearable computing device, first proximity data generated by a proximity sensor of the wearable computing device during a first period of time. The method may further include activating, via the one or more processors, an impedance sensor of the wearable computing device based, at least in part, on the first proximity data. Further, the method may include obtaining, via the one or more processors, impedance data generated by the impedance sensor during a second period of time occurring after the first period of time. Moreover, the method may include obtaining, via the one or more processors, second proximity data generated by the proximity sensor during the second period of time. Additionally, the method may include determining, via the one or more processors, that the wear state of the wearable computing device corresponds to one of an off-wrist state, an on-wrist with contact state, or an on-wrist without contact state based, at least in part, on the impedance data and the second proximity data.

In another aspect, a wearable computing device is provided. The wearable computing device may include a proximity sensor, an impedance sensor, and one or more processors. The one or more processors may be configured to obtain first proximity data generated by the proximity sensor during a first period of time. The one or more processors may further be configured to activate the impedance sensor based, at least in part, on the first proximity data. Further, the one or more processors may be configured to obtain impedance data generated by the impedance sensor during a second period of time occurring after the first period of time. Moreover, the one or more processors may be configured to obtain second proximity data generated by the proximity sensor during the second period of time. Additionally, the one or more processors may be configured to determine that a wear state of the wearable computing device corresponds to one of an off-wrist state, an on-wrist with contact state, or an on-wrist without contact state based, at least in part, on the impedance data and the second proximity data.

In another aspect, a computer-implemented method for determining a wear state of a wearable computing device is provided. The method may include obtaining, via one or more processors of the wearable computing device, impedance data generated by an impedance sensor of the wearable computing device during a period of time. The method may further include calculating, via the one or more processors, an admittance value of the impedance sensor for the period of time based, at least in part, on the impedance data. Moreover, the method may include determining, via the one or more processors, that the wear state of the wearable computing device for the period of time corresponds to an on-wrist state in which the wearable computing device is being worn by a user or an off-wrist state in which the wearable computing device is not being worn by the user based, at least in part, on the calculated admittance value. Additionally, the method may include performing, via the one or more processors, a control action based, at least in part, on the wear state of the computing device.

In yet another aspect, a computer-implemented method for determining a wear state of a wearable computing device is provided. The method may include obtaining, via one or more processors of the wearable computing device, impedance data generated by an impedance sensor of the wearable computing device during a period of time, where a user is wearing the wearable computing device during the period of time. The method may further include determining, via the one or more processors, that electrodes of the impedance sensor were not in contact with skin of the user for a threshold amount of the period of time based, at least in part, on the impedance data. Additionally, the method may include, responsive to determining that the electrodes of the impedance sensor were not in contact with the skin of the user for the threshold amount of the period of time, performing, via the one or more processors, a control action.

These and other features, aspects, and advantages of various embodiments of the present disclosure will become better understood with reference to the following description and appended claims. The accompanying drawings, which are incorporated in and constitute a part of this specification, illustrate example embodiments of the present disclosure and, together with the description, serve to explain the related principles.

### BRIEF DESCRIPTION OF THE DRAWINGS

Detailed discussion of embodiments directed to one of ordinary skill in the art is set forth in the specification, which makes reference to the appended figures, in which:
FIG. 1 provides a perspective view of a wearable computing device on a wrist of a user according to one embodiment of the present disclosure;
FIG. 2 provides a front perspective view of a wearable computing device according to one embodiment of the present disclosure;
FIG. 3 provides a rear perspective view of the wearable computing device of FIG. 2;
FIG. 4 provides an exploded view of the display of the wearable computing device of FIG. 2;
FIG. 5 provides a schematic diagram of an example set of devices that are able to communicate according to one embodiment of the present disclosure;
FIG. 6 illustrates various controller components of an example system that can be utilized according to one embodiment of the present disclosure;
FIG. 7 illustrates a state diagram illustrating different wear states for a wearable computing device according to the present disclosure;
FIGS. 8A and 8B illustrate a flow diagram of an embodiment of a method detecting a wear state of a wearable computing device according to the present disclosure;
FIG. 9 illustrates a flow diagram of an embodiment of another method for detecting wear state of a wearable computing device according to the present disclosure;
FIG. 10 illustrates a flow diagram of an embodiment of yet another method detecting a wear state of a wearable computing device according to the present disclosure;
FIG. 11 illustrates a flow diagram of an embodiment of a further method detecting a wear state of a wearable computing device according to the present disclosure;
FIG. 12 illustrates a graphical representation of a first example notification for a user of a wearable computing device according to the present disclosure;
FIG. 13 illustrates a graphical representation of a second example notification for a user of a wearable computing device according to the present disclosure;
FIG. 14 illustrates a graphical representation of an example notification flow for a user of a wearable computing device according to the present disclosure;
FIG. 15 illustrates another rear perspective view of the wearable computing device of FIG. 2;
FIG. 16 illustrates a further rear perspective view of the wearable computing device of FIG. 2;
FIG. 17 illustrates an exploded view of the skin-side of the wearable computing device of FIG. 2; and
FIGS. 18A-18C illustrate various views of the skin-side of the wearable computing device of FIG. 2, particularly illustrating the skin-side of the wearable computing device when a tinting element is transparent, partially transparent, and opaque, respectively.

### DETAILED DESCRIPTION

Reference now will be made in detail to embodiments of the invention, one or more examples of which are illustrated in the drawings. Each example is provided by way of explanation of the invention, not limitation of the invention. In fact, it will be apparent to those skilled in the art that various modifications and variations can be made in the present invention without departing from the scope of the appended claims. For instance, features illustrated or described as part of one embodiment can be used with another embodiment to yield a still further embodiment. Thus, it is intended that the present invention covers such modifications and variations as come within the scope of the appended claims.

In general, when a wearable computing device is being improperly worn or when the wearable computing device is not sufficiently configured to differentiate between an on-body condition and an off-body condition, data generated by the wearable computing device may be inaccurate and/or misleading. For instance, when a wearable computing device is worn loosely, there may be inconsistent skin contact or shifting area of skin contact which may cause inaccurate data. Similarly, when a wearable computing device is worn overly tight, reduced perfusion (less fluid passage at the skin where the device is worn) or other undesirable effects may be present, which may cause inaccurate data. When the wearable computing device is improperly positioned (e.g., positioned across the styloid process), insufficient contact with the skin may be present, leading to inaccurate data. Additionally, if one or more proximity sensors of the wearable computing device is configured to detect simple reflection of light, the device may determine an on-wrist condition when in proximity to any reflective surface, including inanimate objects, which may lead to inaccurate data or unintended activation of one or more modes. For instance, if the wearable computing device is in a bag and a proximity sensor detects reflection off the bag or another object in the bag (e.g., keys), the device may inadvertently sense a fall and unnecessarily signal for assistance for the user if the bag is dropped, a payment app may be enabled which may allow for unintended payments, data may be collected that does not match with the user's activity (e.g., sleep, exercise, etc.), and/or the like.

As such, the present disclosure is related to a wearable computing device, particularly a wearable computing device having a body impedance sensor, and a computer-implemented method for determining a wear state of the wearable computing device to accurately determine, based at least in part on body impedance data from the body impedance sensor (hereinafter referred to as "impedance sensor"), when the device is being worn and/or when the device is being worn correctly to ensure that measurements by biometric sensors are accurate for determining biometric information and to confirm when a device is being worn for certain activity modes. In general, impedance sensors measure impedance or admittance (which is the inverse of impedance) of the user's skin using a multi-path electrical sensor. These responses are observed as sensitive electrical changes to skin conductance, and are usually detected using wet or dry electrode systems, such as by using at least two electrodes, wherein skin conductance is calculated using the measured electrical impedance. For instance, the impedance sensors may have resistive and reactive components, which yield a two-dimensional output. Impedance sensors may include electrodermal activity (EDA) sensors, which in one embodiment may be used to continuously collect data when operating.

In one or more embodiments, the impedance sensor is turned on or actively collecting data only when the wearable computing device is potentially in proximity of a user, which saves battery life when the data from the impedance sensor would not be accurate and/or would not be valuable. For instance, the wearable computing device may further have a proximity sensor (e.g., a photoplethysmogram (PPG) sensor and/or a pressure sensor) configured to generate proximity data indicative of proximity of the device to a user. The proximity data may be compared to a proximity threshold to determine when the wearable computing device is potentially being worn by a user. When the wearable computing device is determined to be worn by a user based on the comparison of the proximity data to the proximity threshold, the impedance sensor may be turned on or activated to start collecting data. As long as the wearable computing device is determined to be worn by a user based on the proximity data, the impedance data may be used to determine whether the device is being worn and/or worn properly.

In some embodiments, impedance data may be used with or without proximity data to determine whether the device is being worn and/or worn properly. For instance, in one embodiment, the impedance data is taken over a period of time and an admittance value of the impedance sensor for the period of time is calculated based, at least in part, on the impedance data. Thereafter, a wear state of the wearable computing device for the period of time may be determined based, at least in part, on the admittance value. For instance, it may be determined that the wear state is an on-wrist state, corresponding to the wearable computing device being worn by a user, when the admittance value is within an impedance range, or an off-wrist state, corresponding to the wearable computing device not being worn by the user, when the admittance value is outside of the impedance range.

In some embodiments, the impedance data is taken over a period of time and an amount of time that electrodes of the impedance sensor were in contact with skin of the user during the period of time is determined based, at least in part, on the impedance data. If the amount of time that the electrodes of the impedance sensor were in contact with skin of the user is less than a threshold amount of time, but greater than zero, the wear state may be determined to be an on-wrist without contact state and a notification may be generated prompting a user to tighten a band securing the wearable computing device to the user. If the amount of time that the electrodes of the impedance sensor were in contact with skin of the user is zero, the wear state may be determined to be an off-wrist state. Otherwise, if the amount of time that the electrodes of the impedance sensor were in contact with skin of the user is greater than a threshold amount of time, the wear state may be determined to be an on-wrist with contact state.

In some instances, the shape of the impedance data may be monitored over time to determine contact between a user and the device. For instance, after a user starts wearing the device, moisture (e.g., oils, sweat, etc.) slowly begins to accumulate on the device, which causes the admittance value to also slowly increase over time. In some instances, the increase appears like a logarithmic function in shape. As such, the shape of the impedance data may also indicate whether a user has begun to wear the device.

Further, in some instances, monitored impedance or admittance may be used to improve biometric readings from other sensors. For instance, measured changes (e.g., in magnitude and/or angle) in contact impedance or admittance are related to changes in applied pressure, which could be used to improve estimated heart rate and/or the like. Similarly, a combination of proximity data (e.g., from a PPG sensor) and impedance data may be used to improve biometric readings from other sensors. For example, PPG data may be presented as reflectance plotted against wavelength or a reduction in a pulsatile component (AC or DC) of the impedance data, where a sudden shift in the reflectance and/or a shift in the pulsatile component would be indicative a pressure change.

Additionally, in some aspects, a tinting element over at least some of the sensors of the wearable computing device may be controlled based at least in part on the wear state of the wearable computing device. For instance, if the wearable computing device is determined to be worn, the tinting element may be controlled to be transparent such that the sensors covered by the tinting element are unimpeded by the tinting element. Similarly, if the wearable computing device is determined to be off-wrist, the tinting element may be controlled to be at least semi-transparent or fully opaque to at least partially hide the sensors covered by the tinting element. The tinting element may also be controlled to improve sensing conditions for certain sensors. For instance, if ambient light is too high for PPG data to be accurately taken, then the tinting element may be controlled to slightly tint to reduce the ambient light.

Accordingly, the disclosed methods allow for the wear state of the wearable computing device (which may include a fit of the wearable computing device) to be more accurately determined, which in turn allows adjustments to be requested that may increase the overall accuracy of biometric data taken with the wearable computing device and prevent certain activity modes from being activated when the device is not actually being worn.

With reference now to the Figures, example embodiments of the present disclosure will be discussed in further detail.

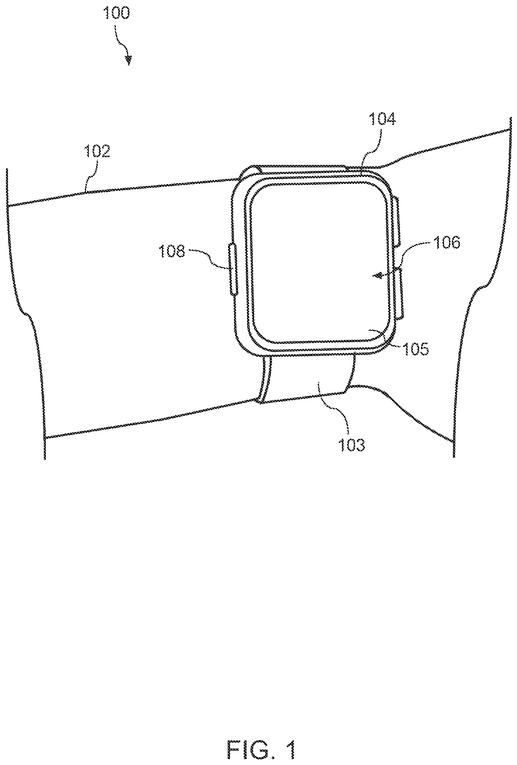

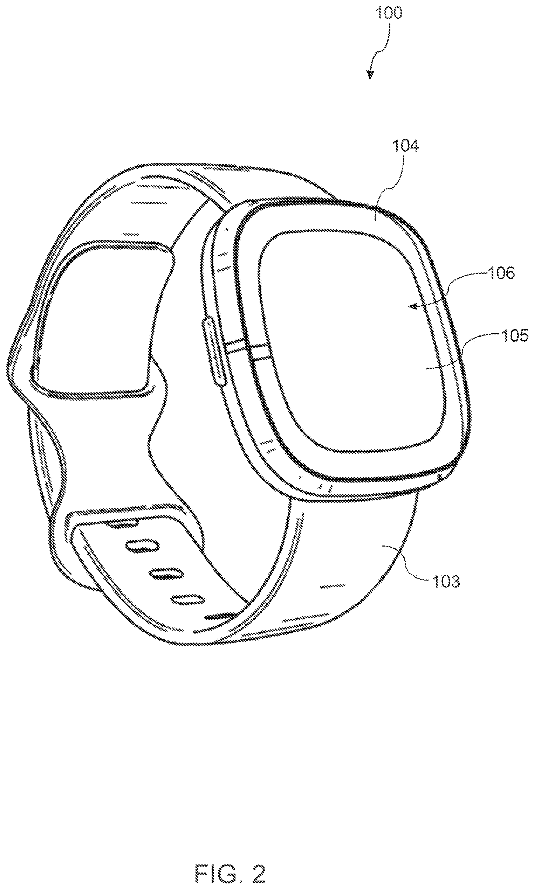

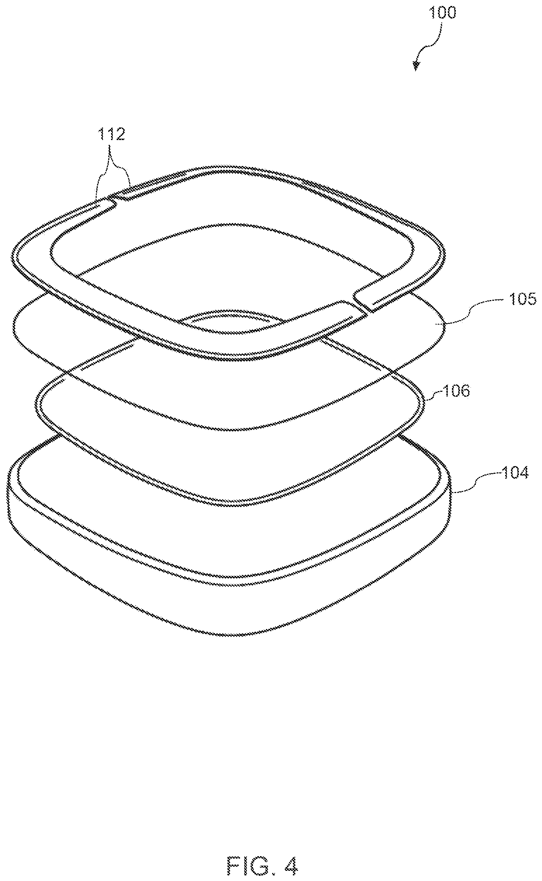

Referring now to the drawings, FIGS. 1-4 illustrate perspective views of a wearable computing device 100 according to the present disclosure. In particular, as shown in FIG. 1, the wearable computing device 100 may be worn on a user's forearm 102 like a wristwatch. Thus, as shown, the wearable computing device 100 may include a wristband 103 for securing the wearable computing device 100 to the user's forearm 102. However, it should be appreciated that the wearable computing device 100 may be worn at any other suitable location by a user, such as, for example, on an ankle. In addition, as shown in FIGS. 1, 2, and 4, the wearable computing device 100 has an outer covering 105 and a housing 104 that contains the electronics associated with the wearable computing device 100. For example, in an embodiment, the outer covering 105 may be constructed of glass, polycarbonate, acrylic, or similar. Further, as shown in FIGS. 1, 2, and 4, the wearable computing device 100 includes an electronic display screen 106 arranged within the housing 104 and viewable through the outer covering 105. Moreover, as shown, the wearable computing device 100 may also include one or more buttons 108 that may be implemented to provide a mechanism to activate various sensors of the wearable computing device 100 to collect certain health data of the user. Moreover, in an embodiment, the electronic display 106 may cover an electronics package (not shown), which may also be housed within the housing 104.

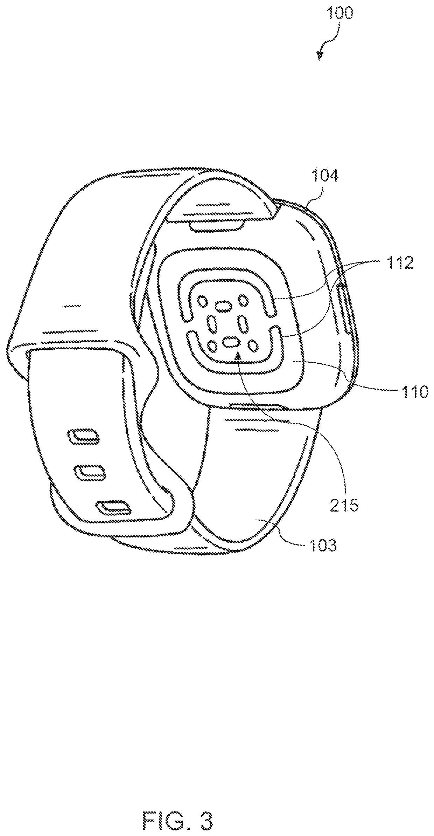

Referring particularly to FIG. 3, the housing 104 of the wearable computing device 100 further includes a dorsal wrist-side face 110 configured to sit against a dorsal wrist of a user when being worn by the user and a plurality of sensor electrodes 112 positioned on the dorsal wrist-side face 110 of the housing 104 so as to maintain skin contact with the user when being worn on the wrist by the user. Thus, in such embodiments, each of the sensor electrodes 112 may be configurable to measure, at least, electrical impedance of the user at a location of the skin contact on the dorsal wrist. Accordingly, in one or more embodiments, one or more (or all) of the plurality of sensor electrodes 112 may be impedance sensor electrodes. In some embodiments, the wearable computing device 100 may also include at least one additional biometric sensor electrode in addition to the impedance sensor electrodes. In such embodiments, the additional biometric sensor electrode 112 may include one or more temperature sensors (such as an ambient temperature sensor or a skin temperature sensor), a humidity sensor, a light sensor, a pressure sensor, a microphone, an optical sensor, or a photoplethysmography (PPG) sensor.

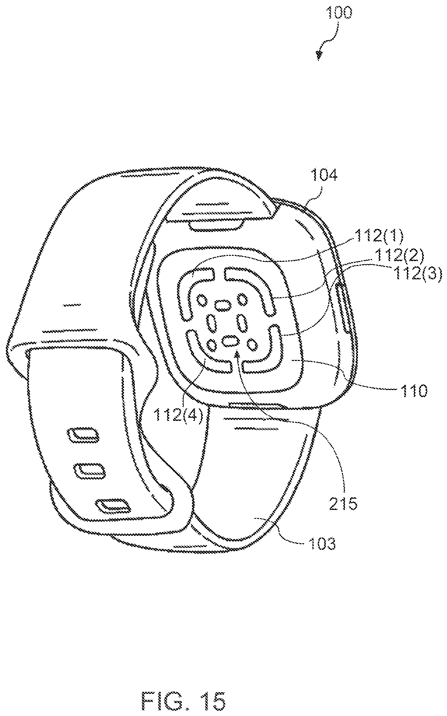

The sensor electrodes 112 may include at least two electrodes spaced apart from each other such that the sensor electrodes 112 are less susceptible to being bridged by sweat and/or the like. For instance, in some embodiments, the wearable computing device 100 may further include an optics package 215 for measuring data for one or more metrics of a human body, such as for a person wearing the wearable computing device 100. In some instances, the sensor electrodes 112 may generally be arranged around the optics package 215. In one embodiment, as shown in FIG. 15, the sensor electrodes 112 may be arranged around the optics package 215 such that the sensor electrodes 112 may be used to indicate partial seating of the wearable computing device 100. For instance, the sensor electrodes 112 may include a first sensor electrode 112(1), a second sensor electrode 112(2), a third sensor electrode 112(3), a fourth sensor electrode 112(4), where each electrode 112(1)-112(4) is associated with a given position around the optics package 215 such that it is easier to determine partial un-seatings. For example, if the first and second sensor electrodes 112(1), 112(2) have readings indicative of contact, but the third and fourth sensor electrodes 112(3), 112(4) do not have readings indicative of contact, the wearable computing device 100 may be determined to be partially unseated with the upper portion (associated with the first and second sensor electrodes 112(1), 112(2)) being seated and the lower portion (associated with the third and fourth sensor electrodes 112(3), 112(4)) being unseated. While only four sensor electrodes 112(1)-112(4) are shown, it should be appreciated that any number of electrodes may be used, and further, that each electrode may be further segmented into multiple sensing areas.

Further, the sensor electrodes 112 described herein may be constructed of any suitable material. For example, in an embodiment, the sensor electrodes 112 described herein may be constructed of stainless steel, graphene, or any other material having a suitable conductivity and/or corrosion resistance and may have an optional PVD coating, that may be 1-micrometer thick titanium nitride. In such embodiments, the PVD coating may provide a desired color to the sensor electrodes 112, thereby preventing oxidation beyond what the stainless steel already provides, and also increases durability.

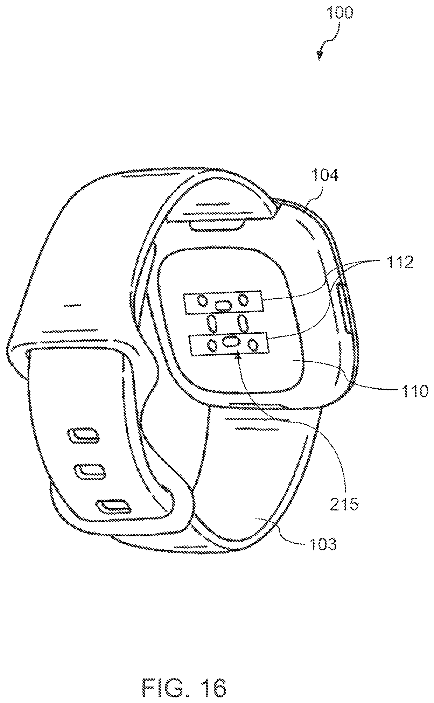

In additional embodiments, PVD and surface finish can be used to increase/decrease moisture retention, which affects the impedance signal and user comfort. In particular embodiments, the sensor electrodes 112 may be formed of an alloy of tin and nickel (TiN) with a shiny or mirror surface finish. Moreover, in an embodiment, the sensor electrodes 112 may be constructed of a hydrophobic material or a transparent material. For instance, as shown in FIG. 16, the sensor electrodes 112 are made of a transparent material (e.g., made from indium tin oxide, fluoro tin oxide (FTO), and/or the like) and are arranged atop the optics package 215. In some embodiments, it may be preferred for the sensor electrodes 112 to be formed from FTO for at least the reason that the FTO may be particularly resilient against general wear, scratching, and/or the like, as the sensor electrodes 112 will be exposed for contact with skin.

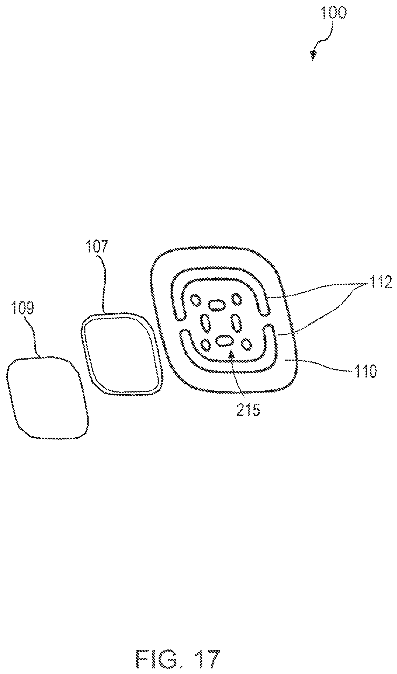

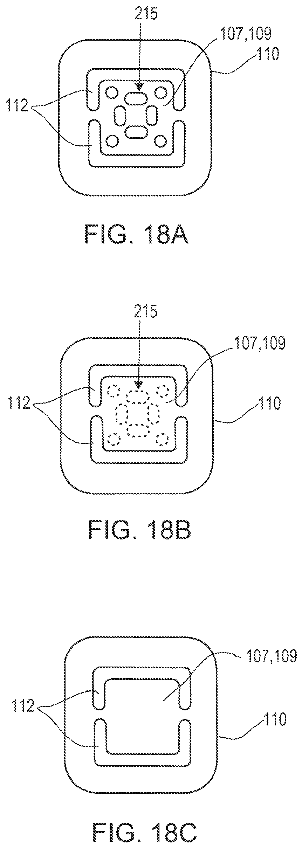

In some embodiments, the wearable computing device 100 may further include one or more tinting elements that may selectively hide one or more of the sensing components on the dorsal wrist-side face 110. For instance, as shown in FIGS. 17-18C, a tinting element 107 may be sandwiched between a protective layer or cover 109 (e.g., glass, resin, plastic, etc.) and at least the optics package 215 on the dorsal wrist-side face 110 of the wearable computing device 100. The tinting element 107 may be a guest host liquid crystal (GHLC) or electrochemical element that is controllable to change between a range of transparencies. For instance, different levels of power or energy supplied to the tinting element 107 may change the amount of transparency of the tinting element 107. For example, as shown in FIG. 18A, the tinting element 107 may be transparent when supplied a first amount of electricity such that the optics package 215 is unobstructed and may operate normally, partially transparent or diffusing (FIG. 18B) when supplied a lower amount of electricity such that the optics package 215 is partially visible and potentially partially operational, or opaque (FIG. 18C) when not supplied electricity such that the optics package 215 is completely hidden and not operational. In the partially transparent state, the lights emitted by the optics package 215 may be at least partially diffused such that the edges of the sensors of the optics package 215 are less visible and the overall appearance is more appealing. In some instances, it may be preferred for the tinting element 107 to be completely transparent when the wearable computing device 100 is detected as being worn, such that the data generated by the optics package 215 are more accurate, and partially transparent or opaque when not worn to at least partially hide the optics package 215. However, in some instances, it may be desirable for the tinting element 107 to be slightly less than fully transparent when worn, such as when ambient lighting is too bright, to improve the readings of certain sensors (e.g., PPG sensor(s) 215). The level of tint between the transparent and opaque states may thus be determined based at least in part on the wear state of the wearable computing device 100 (e.g., determined based on pressure, proximity, and/or the like) and/or ambient lighting.

It should be appreciated that the tinting element 107 may provide global or pixelated tinting of the covered area. Pixelated tinting may particularly allow for increased sensor performance and layouts, particularly for PPG sensors. It should additionally be appreciated that the tinting element 107 may instead be configured to be transparent when unpowered and slightly transparent or opaque when powered.

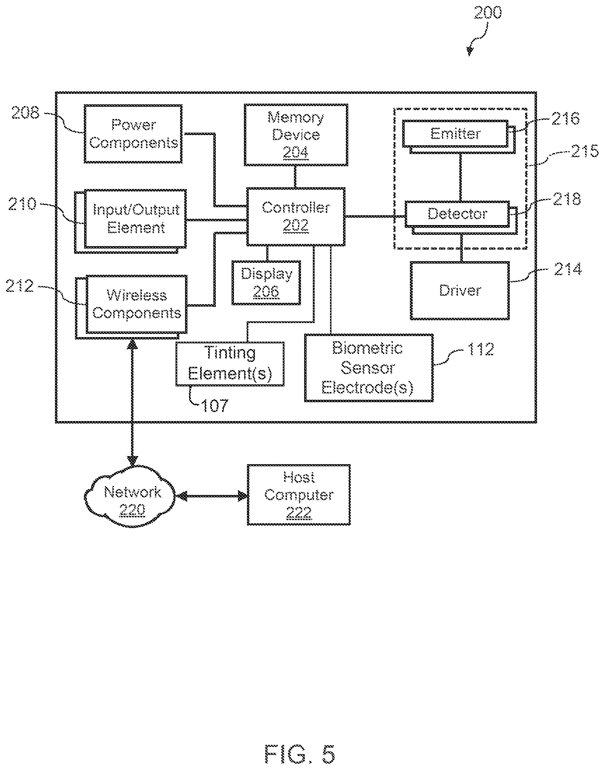

Referring now to FIG. 5, components of an example system 200 of the wearable computing device 100 that can be utilized in accordance with various embodiments are illustrated. In particular, as shown, the system 200 may also include at least one controller 202 communicatively coupled to the plurality of sensor electrodes 112. Moreover, in an embodiment, the controller(s) 202 may be a central processing unit (CPU) or graphics processing unit (GPU) for executing instructions that can be stored in a memory device 204, such as flash memory or DRAM, among other such options.

For example, in an embodiment, the memory device 204 may include RAM, ROM, FLASH memory, or other non-transitory digital data storage, and may include a control program comprising sequences of instructions which, when loaded from the memory device 204 and executed using the controller(s) 202, cause the controller(s) 202 to perform the functions that are described herein. As would be apparent to one of ordinary skill in the art, the system 200 can include many types of memory, data storage, or computer-readable media, such as data storage for program instructions for execution by the controller or any suitable processor. The same or separate storage can be used for images or data, a removable memory can be available for sharing information with other devices, and any number of communication approaches can be available for sharing with other devices.

In addition, as shown, the system 200 includes any suitable display 206, such as a touch screen, organic light emitting diode (OLED), or liquid crystal display (LCD), although devices might convey information via other means, such as through audio speakers, projectors, or casting the display or streaming data to another device, such as a mobile phone, wherein an application on the mobile phone displays the data.

The system 200 may also include one or more wireless components 212 operable to communicate with one or more electronic devices within a communication range of the particular wireless channel. The wireless channel can be any appropriate channel used to enable devices to communicate wirelessly, such as Bluetooth, cellular, NFC, Ultra-Wideband (UWB), or Wi-Fi channels. It should be understood that the system 200 can have one or more conventional wired communications connections as known in the art.

The system 200 also includes one or more power components 208, such as may include a battery operable to be recharged through conventional plug-in approaches, or through other approaches such as capacitive charging through proximity with a power mat or other such device. In further embodiments, the system 200 can also include at least one additional I/O device 210 able to receive conventional input from a user. This conventional input can include, for example, a push button, touch pad, touch screen, wheel, joystick, keyboard, mouse, keypad, or any other such device or element whereby a user can input a command to the system 200. In another embodiment, the I/O device(s) 210 may be connected by a wireless infrared or Bluetooth or other link as well in some embodiments. In some embodiments, the system 200 may also include a microphone or other audio capture element that accepts voice or other audio commands. For example, in particular embodiments, the system 200 may not include any buttons at all, but might be controlled only through a combination of visual and audio commands, such that a user can control the wearable computing device 100 without having to be in contact therewith. In certain embodiments, the I/O elements 210 may also include one or more of the sensor electrodes 112 described herein, optical sensors, barometric sensors (e.g., altimeter, etc.), and the like.

Still referring to FIG. 5, the system 200 may also include a driver 214 and at least some combination of one or more emitters 216 and one or more detectors 218 (referred to herein as an optics package 215) for measuring data for one or more metrics of a human body, such as for a person wearing the wearable computing device 100. In such embodiments, as described above with reference to FIG. 3, for example, the optics package 215 may be arranged within the housing 104 and at least partially exposed through the dorsal wrist-side face 110 of the housing 104. Thus, as shown and further explained herein, the sensor electrodes 112 may be positioned around the optics package 215 on the wrist-side face 110 of the housing 104. In alternative embodiments, the various components of the optics package 215 may be positioned around the sensor electrodes 112 and/or in another other suitable configuration such as adjacent to, interspersed with, surrounded by, or on top of the optics package 215. In certain embodiments, for example, wherein the sensor electrodes 112 are transparent, the sensor electrodes 112 may be arranged atop the optics package 215.

In some embodiments, the system 200 may include at least one imaging element, such as one or more cameras that are able to capture images of the surrounding environment and that are able to image a user, people, or objects in the vicinity of the device. The imaging element can include any appropriate technology, such as a CCD image capture element having a sufficient resolution, focal range, and viewable area to capture an image of the user when the user is operating the device. Further image capture elements may also include depth sensors. Methods for capturing images using a camera element with a computing device are well known in the art and will not be discussed herein in detail. It should be understood that image capture can be performed using a single image, multiple images, periodic imaging, continuous image capturing, image streaming, etc. Further, the system 200 can include the ability to start and/or stop image capture, such as when receiving a command from a user, application, or other device.

The emitters 216 and detectors 218 of FIG. 5 may also be capable of being used, in one example, for obtaining optical PPG measurements. Some PPG technologies rely on detecting light at a single spatial location, adding signals taken from two or more spatial locations, or an algorithmic combination thereof. Both of these approaches result in a single spatial measurement from which the heart rate (HR) estimate (or other physiological metrics) can be determined. In some embodiments, a PPG device employs a single light source coupled to a single detector (i.e., a single light path). Alternatively, a PPG device may employ multiple light sources coupled to a single detector or multiple detectors (i.e., two or more light paths). In other embodiments, a PPG device employs multiple detectors coupled to a single light source or multiple light sources (i.e., two or more light paths). In some cases, the light source(s) may be configured to emit one or more of green, red, infrared (IR) light, as well as any other suitable wavelengths in the spectrum (such as long IR for metabolic monitoring). For example, a PPG device may employ a single light source and two or more light detectors each configured to detect a specific wavelength or wavelength range. In some cases, each detector is configured to detect a different wavelength or wavelength range from one another. In other cases, two or more detectors are configured to detect the same wavelength or wavelength range. In yet another case, one or more detectors configured to detect a specific wavelength or wavelength range different from one or more other detectors). In embodiments employing multiple light paths, the PPG device may determine an average of the signals resulting from the multiple light paths before determining an HR estimate or other physiological metrics.

Moreover, in an embodiment, the emitters 216 and detectors 218 may be coupled to the controller 202 directly or indirectly using driver circuitry by which the controller 202 may drive the emitters 216 and obtain signals from the detectors 218. The host computer 222 can communicate with the wireless networking components 212 via the one or more networks 220, which may include one or more local area networks, wide area networks, UWB, and/or internetworks using any of terrestrial or satellite links. In some embodiments, the host computer 222 executes control programs and/or application programs that are configured to perform some of the functions described herein.

In some embodiments, the system 200 may also include the one or more tinting elements 107 for selectively hiding one or more components on the wearable computing device 100 and be configured to selectively power such tinting element(s) 107 (e.g., via power component(s) 208) to change the tint level of the tinting element(s) 107.

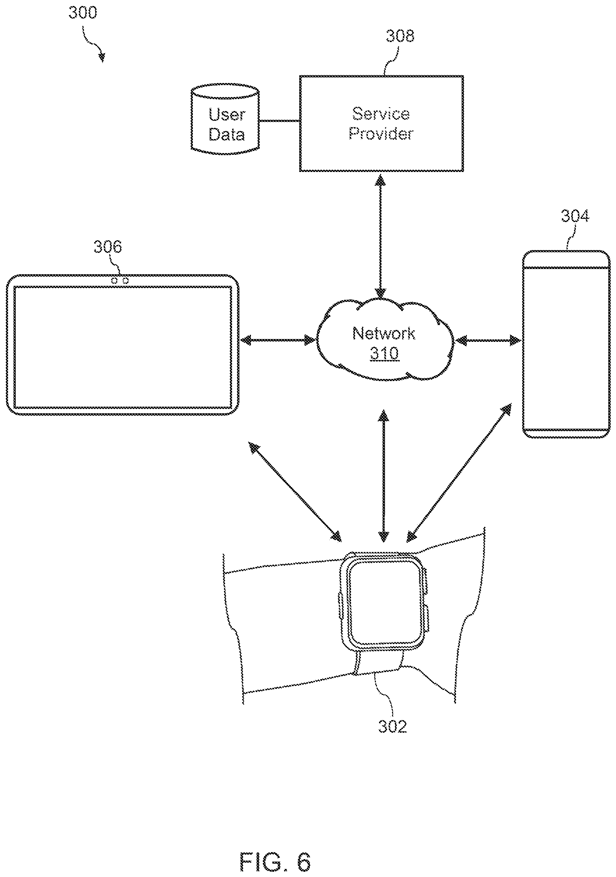

Referring now to FIG. 6, a schematic diagram of an environment 300 in which aspects of various embodiments can be implemented is illustrated. In particular, as shown, a user might have a number of different devices that are able to communicate using at least one wireless communication protocol. For example, as shown, the user might have a smartwatch 302 or fitness tracker (such as wearable computing device 100), which the user would like to be able to communicate with a smartphone 304 and a tablet computer 306. The ability to communicate with multiple devices can enable a user to obtain information from the smartwatch 302, e.g., data captured using a sensor on the smartwatch 302, using an application installed on either the smartphone 304 or the tablet computer 306. The user may also want the smartwatch 302 to be able to communicate with a service provider 308, or other such entity, that is able to obtain and process data from the smartwatch and provide functionality that may not otherwise be available on the smartwatch or the applications installed on the individual devices. In addition, as shown, the smartwatch 302 may be able to communicate with the service provider 308 through at least one network 210, such as the Internet or a cellular network, or may communicate over a wireless connection such as Bluetooth^{®} to one of the individual devices, which can then communicate over the at least one network. There may be a number of other types of, or reasons for, communications in various embodiments.

In addition to being able to communicate, a user may also want the devices to be able to communicate in a number of ways or with certain aspects. For example, the user may want communications between the devices to be secure, particularly where the data may include personal health data or other such communications. The device or application providers may also be required to secure this information in at least some situations. The user may want the devices to be able to communicate with each other concurrently, rather than sequentially. This may be particularly true where pairing may be required, as the user may prefer that each device be paired at most once, such that no manual pairing is required. The user may also desire the communications to be as standards-based as possible, not only so that little manual intervention is required on the part of the user but also so that the devices can communicate with as many other types of devices as possible, which is often not the case for various proprietary formats. A user may thus desire to be able to walk in a room with one device and have such device automatically communicate with another target device with little to no effort on the part of the user. In various conventional approaches, a device will utilize a communication technology such as Wi-Fi to communicate with other devices using wireless local area networking (WLAN). Smaller or lower capacity devices, such as many Internet of Things (IoT) devices, instead utilize a communication technology such as Bluetooth^{®}, and in particular Bluetooth Low Energy (BLE) which has very low power consumption.

In further embodiments, the environment 300 illustrated in FIG. 6 enables data to be captured, processed, and displayed in a number of different ways. For example, data may be captured using sensors on the smartwatch 302, but due to limited resources on the smartwatch 302, the data may be transferred to the smartphone 304 or the service provider 308 (or a cloud resource) for processing, and results of that processing may then be presented back to that user on the smartwatch 302, smartphone 304, and/or another such device associated with that user, such as the tablet computer 306. In at least some embodiments, a user may also be able to provide input such as health data using an interface on any of these devices, which can then be considered when making that determination.

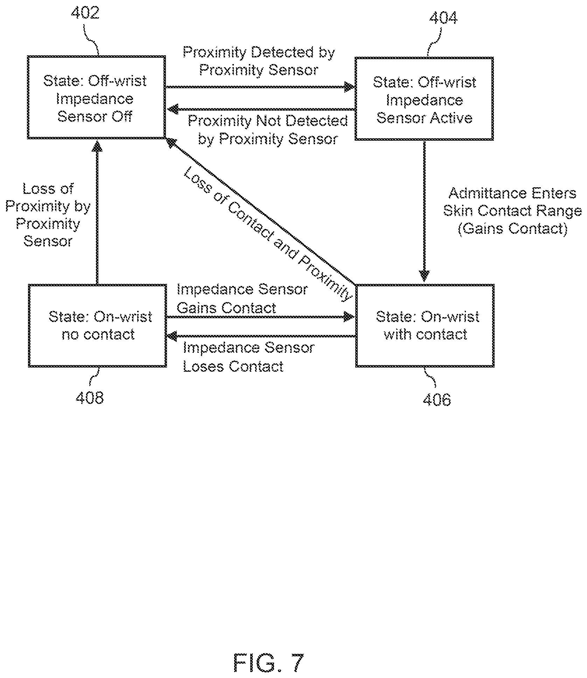

Referring now to FIG. 7, a state diagram illustrating different wear states of a wearable computing device is provided according to the present disclosure. In an embodiment, for example, the wearable computing device may be any suitable wearable computing device, such as the wearable computing device 100 described herein with reference to FIGS. 1-6. Thus, in general, the diagram of the wear states is described herein with reference to the wearable computing device 100 of FIGS. 1-6. However, it should be appreciated that the disclosed wear states may be implemented with any other suitable wearable computing device having any other suitable configurations.

As shown in FIG. 7, the wearable computing device 100 has multiple states depending on the biometric sensor electrodes 112 of the device 100, particularly a proximity sensor and an impedance sensor. For instance, in one embodiment, the wearable computing device 100 has four wear states including: an off-wrist impedance sensor-off state 402, an off-wrist impedance sensor-active state 404, an on-wrist with contact state 406, and an on-wrist no contact state 408.

It should be appreciated that, for purposes of discussion, the proximity sensor will alternatingly be described with reference to a PPG sensor, however, the proximity sensor may be any other suitable proximity sensor or combination of suitable proximity sensors, such as for example, any other suitable optical proximity sensor(s), including infrared sensors. Similarly, in some embodiments, the impedance sensor may be an EDA sensor. However, in other embodiments, the impedance sensor may be any other suitable impedance sensor or combination of impedance sensors, including, but not limited to ultrasound sensors and/or inductive sensors.

In some instances, the wearable computing device 100 defaults to or starts in the off-wrist impedance sensor-off state 402. In the off-wrist impedance sensor-off state 402, the impedance sensor (e.g., EDA sensor) is off and the proximity sensor (e.g., PPG sensor) is on. The data generated by the PPG sensor in the off-wrist impedance sensor-off state 402 indicates an amount of reflected light detected, which is indicative of a proximity of the PPG sensor, and thus, the wearable computing device 100, to an object (e.g., a user). The PPG data is compared to a PPG threshold (e.g., indicative of proximity) to determine whether the wearable computing device 100 is potentially being worn by a user. When the comparison of the PPG data to the PPG threshold indicates that the wearable computing device 100 is not close to an object (e.g., PPG data is less than the PPG threshold or proximity determined from PPG data is greater than a threshold proximity), it is determined that the wearable computing device 100 is not being worn by the user, and the impedance sensor remains in an off state. In some instances, when the device 100 is in the off-wrist impedance sensor-off state 402, certain features of the wearable computing device 100 may be disabled or not allowed to be enabled, such as a workout mode, a sleep mode, a payment mode, etc. Additionally, or alternatively, as indicated above, when the device 100 is in the off-wrist impedance sensor-off state 402, it may be desirable for a tinting element 107 to be in a fully opaque or semi-transparent state.

When the comparison of the PPG data to the PPG threshold otherwise indicates that the wearable computing device 100 is close to an object (e.g., PPG data is above the PPG threshold or proximity determined from PPG data is less than a proximity threshold), it is determined that the wearable computing device 100 is potentially being worn by a user, and the impedance sensor is turned on and the device 100 is transitioned to one of the other states 404, 406, 408.

Particularly, in one embodiment, when the impedance sensor is first turned on, the device 100 is transitioned initially to the off-wrist impedance sensor-active state 404. In the off-wrist impedance sensor-active state 404, the PPG sensor and the impedance sensor are both on. The data generated by the impedance sensor in the off-wrist impedance sensor-active state 404 (e.g. admittance value) is compared to an impedance range (e.g., range of admittance values associated with skin contact of the EDA sensor) to confirm whether the impedance sensor is contacting a user, and thus, whether the device 100 is actually being worn by a user. If the data generated by the impedance sensor in the off-wrist impedance sensor-active state 404 is within the impedance range, the device 100 is determined or confirmed to be worn by a user and the device 100 is transitioned to the on-wrist with contact state 406. Otherwise, if the data generated by the impedance sensor in the off-wrist impedance sensor-active state 404 is not within the impedance range, it is determined or confirmed that the device 100 is not being worn and the device 100 remains in the off-wrist impedance sensor-active state 404. In some embodiments, the shape of the impedance data may additionally, or alternatively, be monitored over time to determine whether the impedance sensor is contacting a user. For instance, after a user starts wearing the device, moisture (e.g., oils, sweat, etc.) slowly begins to accumulate on the device, which causes the admittance value to also slowly increase over time. In some instances, the increase appears like a logarithmic function in shape. As such, the shape of the impedance data generated by the impedance sensor in the off-wrist impedance sensor-active state 404 may be compared to an expected shape to determine whether a user has begun to wear the device 100.

In some instances, when the device 100 in the off-wrist impedance sensor-active state 404, certain features of the wearable computing device 100 may still be disabled or not allowed to be enabled, such as a workout mode, a sleep mode, a payment mode, etc. If the proximity detected by the PPG sensor in the off-wrist impedance sensor-active state 404 indicates that the wearable computing device 100 is no longer close to an object (e.g., PPG data is less than the PPG threshold or proximity determined from PPG data is greater than proximity threshold), it is determined that the device 100 is no longer potentially being worn by a user and the device 100 is transitioned back to the off-wrist impedance sensor-off state 402 and the impedance sensor is turned off or deactivated to save battery, prevent erroneous measurements, prevent unwanted LED flashes, etc. Additionally, or alternatively, as indicated above, when the device 100 is in the off-wrist impedance sensor-active state 404, it may be desirable for a tinting element 107 to be in a fully opaque or semi-transparent state.

When the device 100 is in the on-wrist with contact state 406, the PPG sensor and the impedance sensor are both still on. The data generated by the impedance sensor in the on-wrist with contact state 406 is continued to be compared to the impedance range (e.g., the skin contact range) to determine whether the impedance sensor is still contacting a user and thus, if the device 100 is still being worn by a user. If the data generated by the impedance sensor in the on-wrist with contact state 406 remains within the impedance range, the impedance sensor is still contacting a user and the device 100 is determined to still be worn by a user, so the device 100 remains in the on-wrist with contact state 406. When the device 100 is in the on-wrist with contact state 406, one or more control actions may be performed, such as adjusting the proximity sensor (e.g., adjusting an LED light intensity of the PPG sensor, adjusting a receiver sensitivity, etc. based on characteristics of the user's skin, which may have changed from a previous wear due to activity, sunburn, hygiene, etc.). Similarly, in some instances, when the device 100 is in the on-wrist with contact state 406, certain features of the wearable computing device 100 may be enabled or allowed to be enabled, such as a workout mode, a sleep mode, a payment mode, etc. Moreover, in some instances, when the device 100 is in the on-wrist with contact state 406, the monitored impedance or admittance may be used to improve biometric readings and/or other insights based at least in part on data from other sensors. For instance, measured changes (e.g., in magnitude and/or angle) in contact impedance or admittance are related to changes in applied pressure, which could be used to improve estimated heart rate and/or the like. Similarly, a combination of proximity data (e.g., from a PPG sensor) and impedance data may be used to improve biometric readings from other sensors. For example, PPG data may be presented as reflectance plotted against wavelength or a reduction in a pulsatile component (AC or DC) of the impedance data, where a sudden shift in the reflectance and/or a shift in the pulsatile component would be indicative a pressure change. In some instances, machine learning models may collect data from when the device 100 is in the on-wrist with contact state 406 to improve biometric readings and/or other insights (e.g., battery life) based at least in part on data from other sensors. Additionally, or alternatively, as indicated above, when the device 100 is in the on-wrist with contact state 406, it may be desirable for a tinting element 107 to be in a fully transparent or semi-transparent state.

Otherwise, if the data generated by the impedance sensor in the on-wrist with contact state 406 is no longer within the impedance range, it is determined that impedance sensor is no longer contacting a user and that the device 100 might no longer be worn by a user, so the device 100 is transitioned to the on-wrist no contact state 408. If the proximity detected by the PPG sensor in the on-wrist with contact state 406 indicates that the wearable computing device 100 is no longer close to an object (e.g., PPG data is less than the PPG threshold or proximity determined from PPG data is greater than proximity threshold), it is determined that the device 100 is no longer potentially being worn by a user and the device 100 is transitioned back to the off-wrist impedance sensor off state 402 and the impedance sensor is turned off.

When the device is transitioned to the on-wrist no contact state 408, the PPG sensor and the impedance sensor are both still on. The data generated by the impedance sensor in the on-wrist no contact state 408 is continued to be compared to the impedance range (e.g., the skin contact range) to determine whether the impedance sensor is contacting a user and thus, if the device 100 is still being worn by a user. If the data generated by the impedance sensor in the on-wrist no contact state 408 returns to being within the impedance range, the impedance sensor is again contacting a user and thus, the device 100 is again confirmed as being worn by a user, so the device 100 is transitioned back to the on-wrist with contact state 406. Otherwise, if data generated by the impedance sensor in the on-wrist no contact state 408 remains outside of the impedance range, the impedance sensor is still not contacting a user but may still be worn by a user, so the device 100 remains in the on-wrist no contact state 408. In some instances, as will be described below, a control action may take place when the device 100 is in the on-wrist no contact state 408. For instance, in one instance, the device 100 may prompt a user to adjust the device 100 (e.g., tighten the wristband 103 of the wearable device), and, optionally, device 100 may prompt the user to perform an action to check the fit after adjusting the device 100 (e.g., flick wrist to check that device 100 stays in contact during motion) if certain modes (e.g., exercise, sleep, etc.) are active or enabled from the previous on-wrist with contact state 406 or intended to be activated. In one embodiment, other data generated by the device 100 while in the on-wrist no contact state 408 may be adjusted to account for the device 100 potentially moving out-of-step with the user due to being, e.g., too loose. If the proximity detected by the PPG sensor in the on-wrist no contact state 408 indicates that the wearable computing device 100 is no longer close to an object (e.g., PPG data is less than the PPG threshold or proximity determined from PPG data is greater than proximity threshold), it is determined that the device 100 is no longer potentially being worn by a user and the device 100 is transitioned back to the off-wrist impedance sensor-off state 402 and the impedance sensor is turned off.

As such, it should be appreciated that, in the embodiment shown, the device 100 is only initially confirmed to be worn by a user (i.e., in the on-wrist with contact state 406) when both PPG data is above the PPG threshold and the impedance data is within the impedance range (e.g., above a minimum threshold admittance value and below a maximum threshold admittance value) and continues to be confirmed until the PPG data is below the PPG threshold. It should also be appreciated that using the combination of impedance and PPG data as described prevents the device 100 from unilaterally predicting that the device 100 is in an on-wrist state based on the PPG sensor being close to a reflective surface. Similarly, it should be appreciated that, in some embodiments, using the combination of impedance and PPG data may prevent false off-wrist determinations. For instance, if the PPG data indicates that the proximity is not close enough to be worn by a user, but the impedance data simultaneously indicates contact with a user's skin, the device 100 may be kept in an on-wrist state 406, 408. Additionally, or alternatively, as indicated above, when the device 100 is in the on-wrist no contact state 408, it may be desirable for a tinting element 107 to be in a fully transparent or semi-transparent state.

Moreover, it should be appreciated that, while the device 100 is only shown as being in the on-wrist no contact state 408 after being in the on-wrist with contact state 406, in other embodiments, the device 100 may be in the on-wrist no contact state 408 without first being in the on-wrist with contact state 406. For instance, in some embodiments, the device 100 may be in the on-wrist no contact state 408 without first being in the on-wrist with contact state 406 when the admittance value is non-zero, but otherwise outside of the impedance range. Moreover, it should be appreciated that, in some embodiments, the device 100 may have any other suitable number or combination of states. For instance, in one embodiment, the device 100 only has three states, such as the off-wrist impedance sensor-off state 402, the on-wrist with contact state 406, and the on-wrist no contact state 408, where after determining that the device 100 is within the proximity threshold based on the PPG data and after turning on the impedance sensor, the on-wrist with contact state 406 is determined when the impedance data is within the impedance range or the on-wrist no contact state 408 is determined when the impedance data is outside the impedance range. Additionally, it should be appreciated that the combination of the PPG data and the impedance data may be used to determine a user's skin tone, which may in turn be used to improve determination of the wear status. For instance, different skin tones may each be associated with a different expected reflectance detected by the PPG data for a certain level of contact detected by the impedance sensor (or any other pressure indicating sensor). As such, once the skin tone of the user is determined, the interpretation of the PPG data and other data could be calibrated for such skin tone.

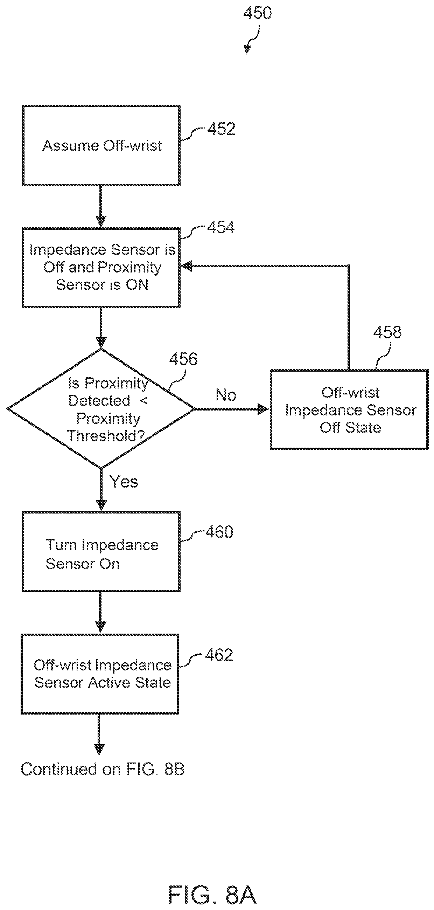

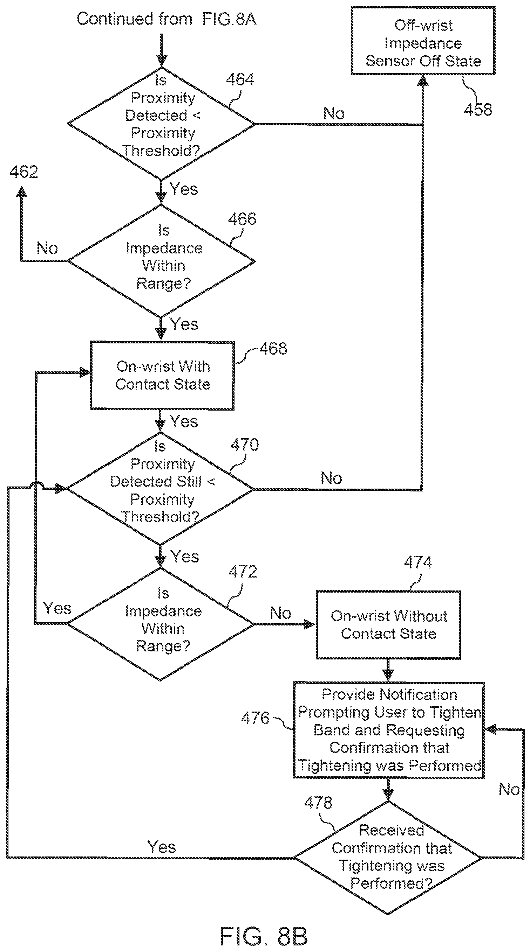

Referring now to FIGS. 8A and 8B, a flow diagram of one embodiment of a method 450 for detecting a wear state of a wearable computing device is provided. In an embodiment, for example, the wearable computing device may be any suitable wearable computing device, such as the wearable computing device 100 described herein with reference to FIGS. 1-6. Thus, in general, the method 450 is described herein with reference to the wearable computing device 100 of FIGS. 1-6 and the wear states 402, 404, 406, 408 described in FIG. 7. However, it should be appreciated that the disclosed method 450 may be implemented with any other suitable wearable computing device having any other suitable configurations and with any other suitable wear states. In addition, although FIGS. 8A and 8B depict steps performed in a particular order for purposes of illustration and discussion, the methods discussed herein are not limited to any particular order or arrangement. One skilled in the art, using the disclosures provided herein, will appreciate that various steps of the methods disclosed herein can be omitted, rearranged, combined, added, and/or adapted in various ways without deviating from the scope of the present disclosure.

Starting in FIG. 8A at step (452) of the method 450, the wearable computing device 100 is assumed to be off-wrist or otherwise not worn by a user. Thus, at step (454) of the method 450, the proximity sensor (e.g., PPG sensor) is on and the impedance sensor (e.g., EDA sensor) is off (or turned off, if not already off), corresponding to the off-wrist impedance sensor-off state 402 described above with reference to FIG. 7. Thereafter, at step (456) of the method 450, the data collected by the PPG sensor is compared to a PPG threshold to determine whether the proximity of the wearable computing device 100 is within a certain proximity of an object (e.g., a user) associated with the wearable computing device 100 being potentially worn by a user. When the PPG data generated by the PPG sensor is less than the PPG threshold (the proximity determined from the PPG data is greater or further than the proximity threshold), it is determined that the wearable computing device 100 is not close to an object and the wearable computing device 100 is not potentially being worn by a user, so the off-wrist impedance sensor-off state is confirmed at step (458) of the method 450 and the impedance sensor remains in an off state. However, when the PPG data generated by the PPG sensor is greater than the PPG threshold (the proximity determined from the PPG data is less than the proximity threshold), it is determined that the wearable computing device 100 is potentially being worn by a user, and the impedance sensor is turned on at step (460) of the method 450. After the impedance sensor is turned on and the device 100 is transitioned to the off-wrist impedance sensor-active state 404 at step (462) of the method 450.

In the off-wrist impedance sensor-active state 404, at step (464) of the method 450 in FIG. 8B, the data collected by the PPG sensor is again compared to a PPG threshold to confirm that the wearable computing device 100 is still within a proximity associated with the wearable computing device 100 being potentially worn by a user. If the PPG data generated by the PPG sensor is less than the PPG threshold (the proximity determined from the PPG data is greater than the proximity threshold), it is determined that the wearable computing device 100 is no longer close to an object and the wearable computing device 100 is no longer potentially being worn by a user, so the device 100 is transitioned back to the off-wrist impedance sensor-off state at step (458) of the method 450 and the impedance sensor is returned to an off state at step (454) (FIG. 8A). Otherwise, if the PPG data generated by the PPG sensor is greater than the PPG threshold (the proximity determined from the PPG data is less than the proximity threshold), it is determined that the wearable computing device 100 is still close to an object and the wearable computing device 100 is still potentially being worn by a user. As long as the wearable computing device 100 is still potentially being worn by a user according to the PPG data at step (464), the impedance data may be evaluated at step (466) of the method 450.

For instance, at step (466), the impedance data (e.g., admittance values) is compared to an impedance range (e.g., admittance values associated with skin contact of the impedance sensor) to determine whether the impedance sensor is in contact with skin of a user. If the data generated by the impedance sensor in the off-wrist impedance sensor-active state 404 is not within the impedance range, it is determined or confirmed that the device 100 is not being worn and the device 100 remains in the off-wrist impedance sensor-active state 404 and the method returns to step (462) (FIG. 8A). Otherwise, if the data generated by the impedance sensor is determined to be within the impedance-related range, the device 100 is determined or confirmed to be worn by a user and the device 100 is transitioned to the on-wrist with contact state 406 at step (468) of the method 450. As such, the impedance data may be used to avoid false on-wrist determinations from PPG data, when the PPG sensor is detecting reflectance off a reflective surface other than skin.

It should be appreciated that, in one embodiment, the steps (464), (466) may instead be performed in reverse order, such that, for a same time instance, if the impedance data is within the impedance-related range, and the PPG data indicates that the proximity is not less than the proximity threshold (that the device 100 is not close enough to a user), the impedance data may override the proximity determined from the PPG data such that the proximity may be determined to still be less than the proximity threshold (that the device 100 is close enough to a user), and one of the on-wrist states 406, 408 is determined instead of the off-wrist impedance sensor-off state 402 for the time instance. Otherwise, if the impedance data is outside the impedance range, and the PPG data indicates that the proximity is not less than the proximity threshold (that the device 100 is not close enough to a user), the impedance data confirms that the proximity determined from the PPG data is not less than the proximity threshold at the time instance, and the off-wrist impedance sensor-off state 402 is returned to at step (458). As such, the impedance data may be used to avoid false off-wrist determinations from PPG data, when the PPG sensor readings are affected by moisture (e.g., sweat, rain, etc.).

Returning to the method 450 illustrated, at step (470), the data collected by the PPG sensor is again compared to a PPG threshold to confirm that the wearable computing device 100 is still within a proximity associated with the wearable computing device 100 being potentially worn by a user. If the PPG data generated by the PPG sensor is less than the PPG threshold (the proximity determined from the PPG data is greater than the proximity threshold), it is determined that the wearable computing device 100 is no longer close to an object and the wearable computing device 100 is no longer potentially being worn by a user, so the device 100 is transitioned back to the off-wrist impedance sensor-off state at step (458) of the method 450 and the impedance sensor is returned to an off state at step (454) (FIG. 8A). Otherwise, if the PPG data generated by the PPG sensor is greater than the PPG threshold (the proximity determined from the PPG data is less than the proximity threshold), it is determined that the wearable computing device 100 is still close to an object and the wearable computing device 100 is still potentially being worn by a user. As long as the wearable computing device 100 is still potentially being worn by a user according to the PPG data at step (464), the impedance data may be evaluated at step (472) of the method 450.

If the data generated by the impedance sensor is still determined to be within the impedance range, the device 100 is determined or confirmed to be worn by a user and the device 100 remains in the on-wrist with contact state 406 and the method returns to step (468). Otherwise, if the data generated by the impedance sensor in the off-wrist impedance sensor-active state 404 is no longer still within the impedance range, the device 100 is transitioned to the on-wrist without contact state 408 (FIG. 7) at step (474) of the method 450.

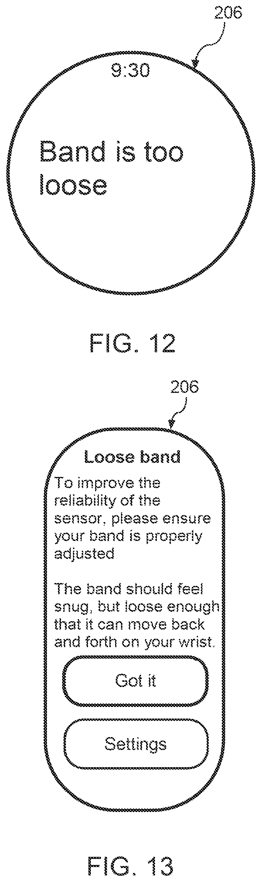

After determining the on-wrist without contact state 408 at step (474), at step (476) of the method 450, a notification is provided that prompts a user to adjust the wearable device (e.g., tighten the wristband 103 of the wearable device). For instance, as shown in FIG. 12, one example of a notification is illustrated where a user interface (e.g., display 206) provides a visual notification indicating to the user that the wearable device 100 needs to be adjusted (e.g., "Band is too loose"). It should be appreciated that the notification may indicate any other suitable message (e.g., "Check fit of band", "Band may be too loose or too tight", etc.). Moreover, it should be appreciated that the notification may be provided in any other suitable way or combination of ways, for instance by haptic feedback, by audio-feedback, and/or the like. In some embodiments, the notification is provided only when a certain threshold of events occurs. For instance, the notification may be provided if the on-wrist without contact state 408 is determined for a particular duration or number of instances over a period of time. In one embodiment, an aggressiveness of the notification may be set based at least in part on the activity mode. For example, if a particular activity mode was initiated, the device may prompt the notification if the on-wrist without contact state 408 is determined for a shorter duration or a smaller number of instances than if a particular activity mode was not initiated.

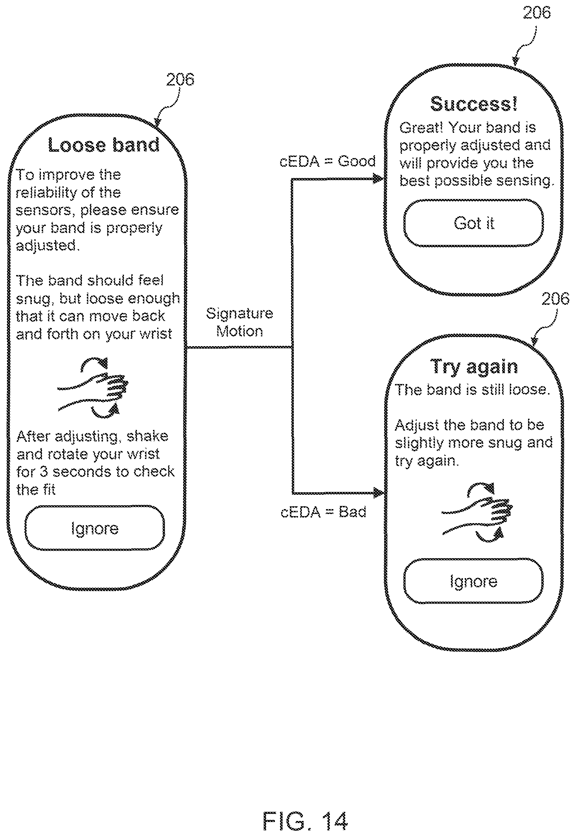

Moreover, in some instances, the notification may also request confirmation that the notification was received. For instance, as shown in FIG. 13, one example of a notification is illustrated where a user interface (e.g., display 206) provides a text-based notification requesting confirmation that the notification was received by interacting with the user interface (e.g., by pressing "Got it"). In one instance, as shown in FIG. 14, the notification may instead, or additionally, include image-based notifications requesting adjusting and/or confirmation. Additionally, in some embodiments, the notification may request confirmation that the adjustment was performed. For example, as also shown in FIG. 14, the notification may prompt the user to perform an action or predefined gesture after adjusting the device 100 (e.g., "After adjusting, shake and rotate your wrist for 3 seconds to check the fit"). It should be appreciated that any other suitable action may be requested, such as tapping the device 100.

When the notification includes a prompt to confirm that the adjustment was performed, at step (478) of the method 450, it is re-determined whether a confirmation was received indicating that the adjustment (e.g., tightening) was performed. For instance, motion data indicative of the user performing the predefined gesture may be received from one or more motion sensors of the device 100 (e.g., the gyroscope, the altimeter, the accelerometer, etc.), and the method may return to step (470) to check if the tightening was sufficient. Otherwise, if the confirmation was not received, the method returns to step (476) and continues to wait.

If after returning to step (470) after adjustment of the device 100 to re-determine the wear state of the wearable computing device, the on-wrist without contact state is again determined at step (474), in some instances, the next notification at step (476) may indicate to the user that the device 100 is still not fitted correctly (e.g., as shown in FIG. 14, "Try again. The band is still loose."). Otherwise, if after returning to step (464) after adjustment of the device 100, the on-wrist with contact state is determined at step (468), a notification may additionally be provided indicating to the user that the adjustment of the device 100 was successful (e.g., as shown in FIG. 14, "Great! Your band is properly adjusted and will provide you the best possible sensing.").

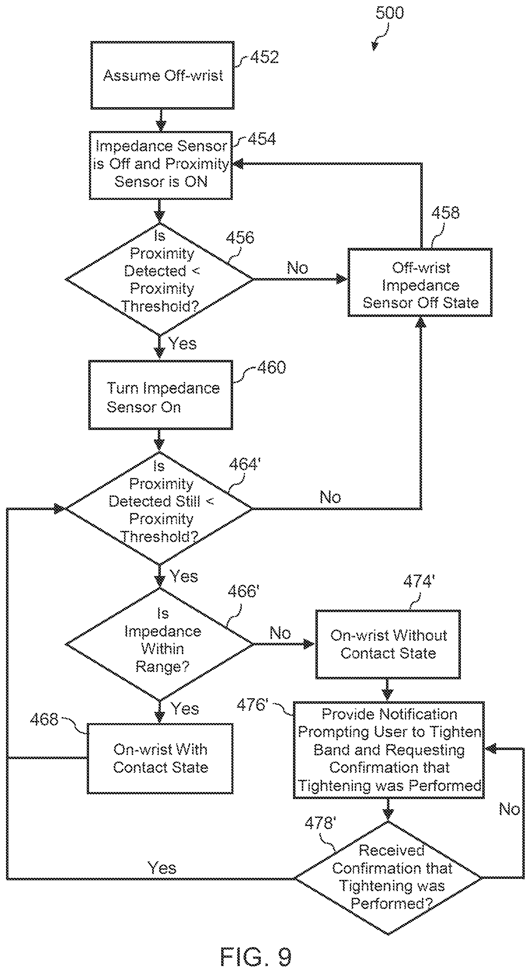

Referring now to FIG. 9, a flow diagram of one embodiment of a method 500 for detecting a wear state of a wearable computing device is provided. In an embodiment, for example, the wearable computing device may be any suitable wearable computing device, such as the wearable computing device 100 described herein with reference to FIGS. 1-6. Thus, in general, the method 500 is described herein with reference to the wearable computing device 100 of FIGS. 1-6 and the wear states 402, 404, 406, 408 described in FIG. 7. However, it should be appreciated that the disclosed method 500 may be implemented with any other suitable wearable computing device having any other suitable configurations and with any other suitable wear states. In addition, although FIG. 9 depicts steps performed in a particular order for purposes of illustration and discussion, the methods discussed herein are not limited to any particular order or arrangement. One skilled in the art, using the disclosures provided herein, will appreciate that various steps of the methods disclosed herein can be omitted, rearranged, combined, added, and/or adapted in various ways without deviating from the scope of the present disclosure.

The method 500 is similar to the method 450 described above in FIGS. 8A and 8B, except that the device 100 may be in the on-wrist no contact state 408 without first being in the on-wrist with contact state 406. For instance, the method 500 has the same steps (452)-(460). After step (460), however, the method 500 does not have step (462). Instead, after step (460), the method 500 moves directly to step (464'), which is the same as step (464) in method 450, and thereafter to step (466'). Like step (466) in method 450, in step (466') of method 500, the impedance data (e.g., admittance values included in the impedance data) is compared to an impedance range (e.g., admittance values associated with skin contact of the EDA sensor) to determine whether the impedance sensor is in contact with skin of a user. If the data generated by the impedance sensor is determined to be within the impedance range, the device 100 is determined or confirmed to be worn by a user and the device 100 is transitioned to the on-wrist with contact state 406 at step (468). However, unlike step (466) in method 450, in step (466') of method 500, if the data generated by the impedance sensor in the off-wrist impedance sensor-active state 404 is not within the impedance range, it is determined or confirmed that the device 100 is not being worn and the device 100 is transitioned to the on-wrist without contact state 404 at step (474'). Thereafter, steps (476') and (478') in method 500 are the same as steps (476) and (478) in method 450, except that after a confirmation that adjustment (e.g., tightening) was performed at step (478') in method 500, the method returns to step 464'.

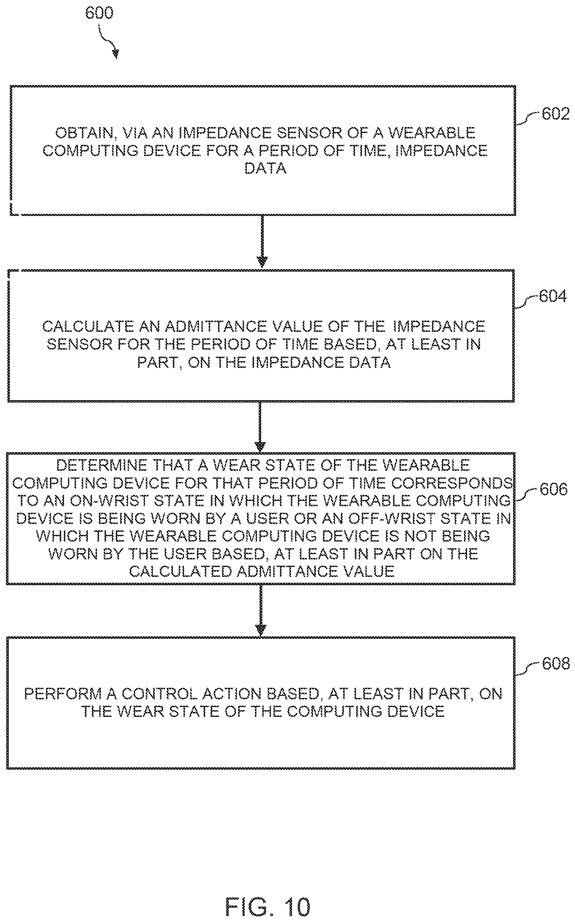

Referring now to FIG. 10, a flow diagram of one embodiment of a method 600 for detecting a wear state of a wearable computing device is provided. In an embodiment, for example, the wearable computing device may be any suitable wearable computing device, such as the wearable computing device 100 described herein with reference to FIGS. 1-6. Thus, in general, the method 600 is described herein with reference to the wearable computing device 100 of FIGS. 1-6 and, optionally, the wear states 402, 404, 406, 408 described in FIG. 7. However, it should be appreciated that the disclosed method 600 may be implemented with any other suitable wearable computing device having any other suitable configurations and with any other suitable wear states. In addition, although FIG. 10 depicts steps performed in a particular order for purposes of illustration and discussion, the methods discussed herein are not limited to any particular order or arrangement. One skilled in the art, using the disclosures provided herein, will appreciate that various steps of the methods disclosed herein can be omitted, rearranged, combined, added, and/or adapted in various ways without deviating from the scope of the present disclosure.

At step (602), the method 600 may include obtaining, via an impedance sensor of a wearable computing device for a period of time, impedance data. For instance, impedance data generated for a period of time by the impedance sensor (e.g., EDA sensor) of the wearable computing device 100 may be obtained. The period of time may be any suitable period of time, such as, for example, about one minute.

Further, at step (604), the method 600 may include calculating an admittance value of the impedance sensor for the period of time based, at least in part, on the impedance data. In general, admittance is the reciprocal of impedance. As such, in one instance, the reciprocal of the impedance data provides admittance values. However, in other embodiments, the impedance data may already be configured to include the admittance values. The admittance value of the impedance sensor for the period of time may be, for example, an average of the admittance values determined from the impedance data.

Moreover, at step (606), the method 600 may include determining that a wear state of the wearable computing device for the period of time corresponds to an on-wrist state in which the wearable computing device is being worn by a user or an off-wrist state in which the wearable computing device is not being worn by the user based, at least in part, on the calculated admittance value. For instance, the admittance value may be compared to an impedance range to determine the wear state of the wearable computing device 100. If the admittance value is within the impedance range (e.g., at or above a minimum threshold admittance value and at or below a maximum threshold admittance value), the wear state corresponds to an on-wrist state in which the wearable computing device 100 is being worn by a user. Otherwise, if the admittance value is outside of the impedance range, the wear state corresponds to an off-wrist state in which the wearable computing device 100 is not being worn by a user. In some instances, a 2D region in a complex admittance space (e.g., based on phasor notation of admittance, with the complex part of the admittance (representative of reactance) being plotted relative to the real part of the admittance (representative of resistance)) may additionally or alternatively be defined that corresponds to human skin contact. When the admittance (e.g., reactance and resistance) from the impedance data falls within the 2D region, the wear state corresponds to an on-wrist state in which the wearable computing device 100 is being worn by a user. Otherwise, if the admittance from the impedance data is outside of the 2D region, the wear state corresponds to an off-wrist state in which the wearable computing device 100 is not being worn by a user. By using such 2D admittance region, materials with a similar overall magnitude of admittance (combination of reactance and resistance) may be prevented from being falsely detected as being skin, as at least one of the reactance or the resistance of the admittance is likely to differ from the reactance or the resistance of skin. In some instances, the total time that the admittance value is within or outside of the impedance range is used to determine the wear state. For instance, if the total time the admittance value is within the impedance range over a given time period is greater than a threshold period of time, the device 100 may be determined to be in an on-wrist state. Conversely, if the total time the admittance value is within the impedance range over a given time period is less than a threshold period of time, the device 100 may be determined to be in an off-wrist state.

Additionally, at step (608), the method 600 may include performing a control action based, at least in part, on the wear state of the computing device. For instance, the control action may include generating a notification indicative of the wear state of the wearable computing device for the period of time. In some instances, the control action may include generating a notification prompting the user to adjust a fit of the wearable computing device 100 (e.g., tighten band 103, rotate band 103, etc.) and/or adjusting date from the sensors when the wear state corresponds to the off-wrist state intermittently, and/or deactivating one or more features (e.g., payment mode) of the wearable computing device 100 when the wear state corresponds to the off-wrist state for a sustained period. In one instance, when the wear state corresponds to the on-wrist state for a sustained period, the control action may include enabling or allowing certain features of the wearable computing device 100 to be used, such as a workout mode, a sleep mode, a payment mode, etc.

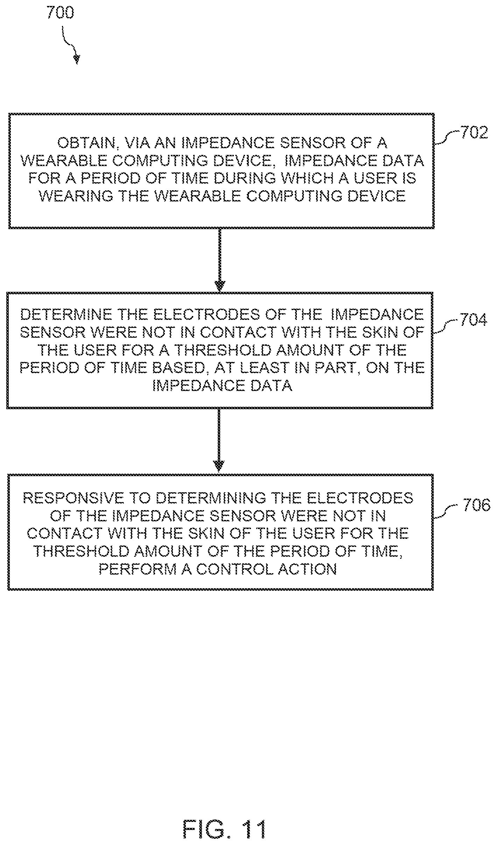

Referring now to FIG. 11, a flow diagram of one embodiment of a method 700 for detecting a wear state of a wearable computing device is provided. In an embodiment, for example, the wearable computing device may be any suitable wearable computing device, such as the wearable computing device 100 described herein with reference to FIGS. 1-6. Thus, in general, the method 700 is described herein with reference to the wearable computing device 100 of FIGS. 1-6 and, optionally, the wear states 402, 404, 406, 408 described in FIG. 7. However, it should be appreciated that the disclosed method 700 may be implemented with any other suitable wearable computing device having any other suitable configurations and with any other suitable wear states. In addition, although FIG. 11 depicts steps performed in a particular order for purposes of illustration and discussion, the methods discussed herein are not limited to any particular order or arrangement. One skilled in the art, using the disclosures provided herein, will appreciate that various steps of the methods disclosed herein can be omitted, rearranged, combined, added, and/or adapted in various ways without deviating from the scope of the present disclosure.

At step (702), the method 700 may include obtaining, via an impedance sensor (e.g., EDA sensor) of a wearable computing device, impedance data for a period of time during which a user is wearing the wearable computing device. For instance, impedance data generated for a period of time by the impedance sensor of the wearable computing device 100 may be obtained. In general, the impedance data includes a plurality of measurements indicative of electrodes of the impedance sensor contacting the skin of the user during the period of time.

Further, at step (704), the method 700 may include determining that electrodes of the impedance sensor were not in contact with skin of the user for a threshold amount of the period of time based, at least in part, on the impedance data. For example, the impedance data only has data points when the impedance electrodes are in contact with the skin. As such, a total number of the plurality of measurements obtained during the period of time may be compared to a minimum threshold number of measurements associated with the threshold amount of the period of time (e.g., a minimum of 100 measurements should be taken within 1 minute, associated with contact between the electrodes and skin for at least 50% of the minute (30 seconds)). If the total number of the plurality of measurements obtained during the period of time is less than the threshold number of measurements, but greater than zero, the electrodes of the impedance sensor are determined to not be in contact with the skin of the user for the threshold amount of the period of time, and the on-wrist without contact state 408 is determined. If the total number of the plurality of measurements obtained during the period of time is less than the threshold number of measurements, particularly if the total number of the plurality of measurements obtained during the period of time is zero, the electrodes of the impedance sensor are determined to not be in contact with the skin of the user at all during the period of time, and an off-wrist state is determined. Otherwise, if the total number of the plurality of measurements obtained during the period of time is greater than the threshold number of measurements, the electrodes of the impedance sensor are determined to be in contact with the skin of the user for the threshold amount of the period of time and the on-wrist with contact state 406 is determined.

Additionally, at step (706), the method 700 may include, responsive to determining the electrodes of the impedance sensor were not in contact with the skin of the user for the threshold amount of the period of time, performing a control action. For instance, when the total number of the plurality of measurements obtained during the period of time is less than the threshold number of measurements, the wearable computing device 100 may determine the device 100 to be improperly fitted to the user and then perform a control action. For example, as indicated above, the device 100 may generate a notification to prompt the user to tighten a band securing the wearable computing device to the user, and the user may be prompted with a notification to adjust the wearable computing device 100 (e.g., tighten the band 103, rotate the band 103, etc.). Similarly, as indicated above, the device 100 may adjust the data collected to account for the device 100 being improperly fitted.

It should be appreciated that the methods 600, 700 may be performed without determining that the proximity of the device 100 is below a proximity threshold based on PPG data from a PPG sensor. However, in some instances, the methods 600, 700 may be performed only when the proximity of the device 100 is determined to be below the proximity threshold based on PPG data from a PPG sensor, such as described with reference to steps (452)-(460) of methods 450, 500.

It should additionally be appreciated that the methods 450, 500, 600, 700, may be performed at the initiation of certain activity modes of the wearable computing device 100. For instance, certain applications (e.g., workout tracking, sleep tracking, meditation, etc.) require more precise data to generate reports, or may have higher subjective value to the user than data during other activities. When the wearable computing device 100 is not properly fitted (e.g., loose), the data generated by the biometric sensors of the wearable computing device 100 may be insufficient (e.g., sporadic or inaccurate) to generate reports. As such, when one of these applications on the wearable computing device 100 is likely to be initiated (such as based on habits of a user, pre-programmed start times, biometric readings, and/or the like) or is explicitly initiated by a user, the methods 450, 500, 600, 700 may be used to ensure that the wearable computing device 100 is on the user and/or that the device 100 is properly fitted to the user. Further, by prompting the user to correct a fit of the wearable computing device 100, instances can be avoided in which the application (e.g., sleep tracking) is unable to generate a report (e.g., sleep score, snore report, etc.) due to insufficient biometric data caused, at least in part, by the wearable computing device 100 not being worn correctly (e.g., loose) on the user's wrist. Data produced by the wearable computing device 100 may also be edited or highlighted to indicate confidence in data depending on the wear status during the activity mode. For instance, a low confidence value for the data may be suggested and/or the data may not be shown when the wear status is the on-wrist without contact state 408, and therefore that the data is of a lower quality and/or less reliable, whereas a higher confidence value for the data may be suggested when the wear status is the on-wrist with contact state 406. Additionally, certain applications, such as payment applications, may require a higher level of certainty that the wearable computing device 100 is being worn by a user to be enabled. As such, it may be beneficial to further require an authorization action by the user with the wearable computing device 100 for payment (e.g., with PIN entry, motion pattern, etc.) if the wear status is one of the on-wrist states 406, 408 and de-authorizing the wearable computing device 100 for payment if the wear status is an off-wrist state 402, 404, etc.

The technology discussed herein makes reference to servers, databases, software applications, and other computer-based systems, as well as actions taken and information sent to and from such systems. The inherent flexibility of computer-based systems allows for a great variety of possible configurations, combinations, and divisions of tasks and functionality between and among components. For instance, processes discussed herein can be implemented using a single device or component or multiple devices or components working in combination. Databases and applications can be implemented on a single system or distributed across multiple systems. Distributed components can operate sequentially or in parallel.

Again, although the above-described methods for determining a wear state of a wearable computing device have been discussed in the context of using a PPG sensor, it should be understood that the methods are not intended to be limited to determining the wear state based on PPG data in conjunction with the impedance data. It should be appreciated that the impedance sensor can be used with any suitable proximity sensor to determine wrist state. For example, in some embodiments, the wearable computing device can include one or more capacitive sensors, ultrasound sensors, inductive sensors, and/or the like configured to determine proximity of the wearable computing device to the user. In such embodiments, the wear state of the wearable computing device can be determined based on the impedance data obtained from the impedance sensor and capacitance data obtained from the one or more capacitive sensors, ultrasound sensors, inductive sensors, and/or the like.

While the present subject matter has been described in detail with respect to various specific example embodiments thereof, each example is provided by way of explanation, not limitation of the disclosure. Those skilled in the art, upon attaining an understanding of the foregoing, can readily produce alterations to, variations of, and equivalents to such embodiments. Accordingly, the subject disclosure does not preclude inclusion of such modifications, variations and/or additions to the present subject matter as would be readily apparent to one of ordinary skill in the art. For instance, features illustrated or described as part of one embodiment can be used with another embodiment to yield a still further embodiment. Thus, it is intended that the present disclosure covers such alterations, variations, and equivalents.

## Claims

1. A computer-implemented method for determining a wear state of a wearable computing device (100), the method comprising:
obtaining, by one or more processors of the wearable computing device (100), first proximity data generated by a proximity sensor of the wearable computing device (100) during a first period of time;
activating, via the one or more processors, an impedance sensor of the wearable computing device (100) based, at least in part, on the first proximity data;
obtaining, via the one or more processors, impedance data generated by the impedance sensor during a second period of time occurring after the first period of time;
obtaining, via the one or more processors, second proximity data generated by the proximity sensor during the second period of time; and
determining, via the one or more processors, that the wear state of the wearable computing device (100) corresponds to one of an off-wrist state (402, 404), an on-wrist with contact state (406), or an on-wrist without contact state (408) based, at least in part, on the impedance data and the second proximity data.

2. The computer-implemented method of claim 1, wherein activating the impedance sensor based on the first proximity data includes:
determining, via the one or more processors, whether the wearable computing device (100) is in proximity to skin of a user based, at least in part, on the first proximity data; and
activating, via the one or more processors, the impedance sensor in response to determining that the wearable computing device (100) is in proximity to the skin of the user based, at least in part, on the first proximity data.

3. The computer-implemented method of claim 2, wherein determining that the wear state of the wearable computing device (100) corresponds to the on-wrist with contact state (406) includes:
determining, via the one or more processors, the wearable computing device (100) is still in proximity to the skin of the user based, at least in part, on the second proximity data;
determining, via the one or more processors, the impedance sensor is contacting the skin of the user based, at least in part, on the impedance data; and
responsive to determining that the wearable computing device (100) is still in proximity to the skin of the user and the impedance sensor is contacting the skin of the user, determining, via the one or more processors, the wear state for the wearable computing device (100) corresponds to the on-wrist with contact state (406),
optionally wherein determining that the impedance sensor is contacting the skin of the user includes determining, via the one or more processors, admittance values included in the impedance data is within a range of admittance values included in the impedance data.

4. The computer-implemented method of claim 2, wherein determining that the wear state of the wearable computing device (100) corresponds to the on-wrist without contact state (408) includes:
determining, via the one or more processors, the wearable computing device (100) is still in proximity to the skin of the user based, at least in part, on the second proximity data;
determining, via the one or more processors, the impedance sensor is not contacting the skin of the user based, at least in part, on the impedance data; and
responsive to determining that the wearable computing device (100) is still in proximity to the skin of the user and the impedance sensor is not contacting the skin of the user, determining, via the one or more processors, the wear state for the wearable computing device (100) corresponds to the on-wrist without contact state (408).

5. The computer-implemented method of claim 4, wherein determining that the impedance sensor is not contacting the skin of the user includes determining, via the one or more processors, admittance values included in the impedance data are outside a range of admittance values indicative of the impedance sensor contacting the skin of the user.

6. The computer-implemented method of claim 4, further comprising:
after determining that the wear state of the wearable computing device (100) corresponds to the on-wrist without contact state (408), generating, via the one or more processors, a notification to prompt the user to tighten a band (103) of the wearable computing device (100).

7. The computer-implemented method of claim 6, wherein notification prompts the user to perform a predefined gesture to confirm tightening of the band (103).

8. The computer-implemented method of claim 7, further comprising:
obtaining, via the one or more processors, motion data generated by one or more motion sensors of the wearable computing device, the motion data being indicative of the user performing the predefined gesture; and
obtaining, via the one or more processors, second impedance data in response to obtaining the motion data indicative of the user performing the predefined gesture, the second impedance data being generated by the impedance sensor;
obtaining, via the one or more processors, third proximity data in response to obtaining the motion data indicative of the user performing the predefined gesture, the third proximity data being generated by the proximity sensor; and
re-determining, via the one or more processors, the wear state of the wearable computing device based, at least in part, on the second impedance data and the third proximity data.

9. The computer-implemented method of claim 4, further comprising, after determining that the wear state of the wearable computing device (100) corresponds to the on-wrist without contact state (408), at least one of:
adjusting data generated by the wearable computing device (100) taken during the on-wrist without contact state (408); or
adjusting a confidence value for the data generated by the wearable computing device (100) taken during the on-wrist without contact state (408).

10. The computer-implemented method of claim 2, wherein determining that the wear state of the wearable computing device (100) corresponds to the off-wrist state (404) includes:
determining, via the one or more processors, the wearable computing device (100) is no longer in proximity to the skin of the user based, at least in part, on the second proximity data;
determining, via the one or more processors, the impedance sensor is not contacting the skin of the user based, at least in part, on the impedance data; and
responsive to determining that the wearable computing device (100) is not still in proximity to the skin of the user and the impedance sensor is not contacting the skin of the user, determining, via the one or more processors, the wear state for the wearable computing device (100) corresponds to an off-wrist state (404).

11. The computer-implemented method of claim 10, further comprising:
deactivating, via the one or more processors, the impedance sensor in response to determining that the wear state for the wearable computing device (100) corresponds to the off-wrist state (402) and is no longer in proximity to the skin of the user.

12. A wearable computing device (100) comprising:
a proximity sensor;
an impedance sensor; and
one or more processors configured to:
obtain first proximity data generated by the proximity sensor during a first period of time;
activate the impedance sensor based, at least in part, on the first proximity data;
obtain impedance data generated by the impedance sensor during a second period of time occurring after the first period of time;
obtain second proximity data generated by the proximity sensor during the second period of time; and
determine that a wear state of the wearable computing device (100) corresponds to one of an off-wrist state (402, 404), an on-wrist with contact state (406), or an on-wrist without contact state (408) based, at least in part, on the impedance data and the second proximity data.

13. The wearable computing device (100) of claim 12, wherein the proximity sensor includes one or more ultrasound sensors, inductive sensors, or both, or an optical sensor.

14. The wearable computing device (100) of claim 12, further comprising:
a cover (105; 109); and
a tinting element (107) between the cover (105; 109) and at least the proximity sensor,
wherein the one or more processors is further configured to control the tinting element (107) to change between a transparent state and an opaque state based at least in part on the wear state.

15. The wearable computing device (100) of claim 12, wherein the impedance sensor comprises at least a first electrode (112) and a second electrode (112) spaced apart from each other,
wherein the impedance data is indicative of contact at the first electrode (112) and contact at the second electrode (112), optionally
wherein the proximity sensor is between the first and second electrodes (112) or
the first and second electrodes (112) are made of a transparent material, the first and second electrodes (112) being positioned above the proximity sensor.

## Patentansprüche

1. Computerimplementiertes Verfahren zur Bestimmung eines Tragezustands einer tragbaren Rechenvorrichtung (100), wobei das Verfahren umfasst:
Erhalten, durch einen oder mehrere Prozessoren der tragbaren Rechenvorrichtung (100), von ersten Näherungsdaten, die durch einen Näherungssensor der tragbaren Rechenvorrichtung (100) während einer ersten Zeitspanne erzeugt werden;
Aktivieren, über den einen oder die mehreren Prozessoren, eines Impedanzsensors der tragbaren Rechenvorrichtung (100) basierend, zumindest teilweise, auf den ersten Näherungsdaten;
Erhalten, über den einen oder die mehreren Prozessoren, von Impedanzdaten, die durch den Impedanzsensor während einer zweiten Zeitspanne erzeugt werden, die nach der ersten Zeitspanne auftritt;
Erhalten, über den einen oder die mehreren Prozessoren, von zweiten Näherungsdaten, die durch den Näherungssensor während der zweiten Zeitspanne erzeugt werden; und
Bestimmen, über den einen oder die mehreren Prozessoren, dass der Tragezustand der tragbaren Rechenvorrichtung (100) einem von einem Zustand (402, 404) nicht am Handgelenk, einem Zustand (406) am Handgelenk mit Kontakt oder einem Zustand (408) am Handgelenk ohne Kontakt entspricht, basierend, zumindest teilweise, auf den Impedanzdaten und den zweiten Näherungsdaten.

2. Computerimplementiertes Verfahren nach Anspruch 1, wobei Aktivieren des Impedanzsensors basierend auf den ersten Näherungsdaten beinhaltet:
Bestimmen, über den einen oder die mehreren Prozessoren, ob sich die tragbare Rechenvorrichtung (100) in der Nähe von Haut eines Benutzers befindet, basierend, zumindest teilweise, auf den ersten Näherungsdaten; und
Aktivieren, über den einen oder die mehreren Prozessoren, des Impedanzsensors als Reaktion auf Bestimmen, dass sich die tragbare Rechenvorrichtung (100) in der Nähe der Haut des Benutzers befindet, basierend, zumindest teilweise, auf den ersten Näherungsdaten.

3. Computerimplementiertes Verfahren nach Anspruch 2, wobei Bestimmen, dass der Tragezustand der tragbaren Rechenvorrichtung (100) dem Zustand (406) am Handgelenk mit Kontakt entspricht, beinhaltet:
Bestimmen, über den einen oder die mehreren Prozessoren, dass sich die tragbare Rechenvorrichtung (100) immer noch in der Nähe der Haut des Benutzers befindet, basierend, zumindest teilweise, auf den zweiten Näherungsdaten;
Bestimmen, über den einen oder die mehreren Prozessoren, dass der Impedanzsensor die Haut des Benutzers kontaktiert, basierend, zumindest teilweise, auf den Impedanzdaten; und
als Reaktion auf Bestimmen, dass sich die tragbare Rechenvorrichtung (100) immer noch in der Nähe der Haut des Benutzers befindet und der Impedanzsensor die Haut des Benutzers kontaktiert,
Bestimmen, über den einen oder die mehreren Prozessoren, dass der Tragezustand für die tragbare Rechenvorrichtung (100) dem Zustand (406) am Handgelenk mit Kontakt entspricht,
wobei optional Bestimmen, dass der Impedanzsensor die Haut des Benutzers kontaktiert, Bestimmen, über den einen oder die mehreren Prozessoren, beinhaltet, dass Admittanzwerte, die in den Impedanzdaten beinhaltet sind, innerhalb eines Bereichs von Admittanzwerten liegen, die in den Impedanzdaten beinhaltet sind.

4. Computerimplementiertes Verfahren nach Anspruch 2, wobei Bestimmen, dass der Tragezustand der tragbaren Rechenvorrichtung (100) dem Zustand (408) am Handgelenk ohne Kontakt entspricht, beinhaltet:
Bestimmen, über den einen oder die mehreren Prozessoren, dass sich die tragbare Rechenvorrichtung (100) immer noch in der Nähe der Haut des Benutzers befindet, basierend, zumindest teilweise, auf den zweiten Näherungsdaten;
Bestimmen, über den einen oder die mehreren Prozessoren, dass der Impedanzsensor die Haut des Benutzers nicht kontaktiert, basierend, zumindest teilweise, auf den Impedanzdaten; und
als Reaktion auf Bestimmen, dass sich die tragbare Rechenvorrichtung (100) immer noch in der Nähe der Haut des Benutzers befindet und der Impedanzsensor die Haut des Benutzers nicht kontaktiert, Bestimmen, über den einen oder die mehreren Prozessoren, dass der Tragezustand für die tragbare Rechenvorrichtung (100) dem Zustand (408) am Handgelenk ohne Kontakt entspricht.

5. Computerimplementiertes Verfahren nach Anspruch 4, wobei Bestimmen, dass der Impedanzsensor die Haut des Benutzers nicht kontaktiert, Bestimmen, über den einen oder die mehreren Prozessoren, beinhaltet, dass Admittanzwerte, die in den Impedanzdaten beinhaltet sind, außerhalb eines Bereichs von Admittanzwerten liegen, die anzeigen, dass der Impedanzsensor die Haut des Benutzers kontaktiert.

6. Computerimplementiertes Verfahren nach Anspruch 4, ferner umfassend:
nach Bestimmen, dass der Tragezustand der tragbaren Rechenvorrichtung (100) dem Zustand (408) am Handgelenk ohne Kontakt entspricht, Erzeugen, über den einen oder die mehreren Prozessoren, einer Benachrichtigung, um den Benutzer aufzufordern, ein Band (103) der tragbaren Rechenvorrichtung (100) festzuziehen.

7. Computerimplementiertes Verfahren nach Anspruch 6, wobei die Benachrichtigung den Benutzer auffordert, eine vordefinierte Geste durchzuführen, um Festziehen des Bandes (103) zu bestätigen.

8. Computerimplementiertes Verfahren nach Anspruch 7, ferner umfassend:
Erhalten, über den einen oder die mehreren Prozessoren, von Bewegungsdaten, die durch einen oder mehrere Bewegungssensoren der tragbaren Rechenvorrichtung erzeugt werden, wobei die Bewegungsdaten anzeigend für den Benutzer sind, der die vordefinierte Geste durchführt; und
Erhalten, über den einen oder die mehreren Prozessoren, von zweiten Impedanzdaten als Reaktion auf Erhalten der Bewegungsdaten, die anzeigend für den Benutzer sind, der die vordefinierte Geste durchführt, wobei die zweiten Impedanzdaten durch den Impedanzsensor erzeugt werden;
Erhalten, über den einen oder die mehreren Prozessoren, von dritten Näherungsdaten als Reaktion auf Erhalten der Bewegungsdaten, die anzeigend für den Benutzer sind, der die vordefinierte Geste durchführt, wobei die dritten Näherungsdaten durch den Näherungssensor erzeugt werden; und
erneutes Bestimmen, über den einen oder die mehreren Prozessoren, des Tragezustands der tragbaren Rechenvorrichtung basierend, zumindest teilweise, auf den zweiten Impedanzdaten und den dritten Näherungsdaten.

9. Computerimplementiertes Verfahren nach Anspruch 4, ferner umfassend, nach Bestimmen, dass der Tragezustand der tragbaren Rechenvorrichtung (100) dem Zustand (408) am Handgelenk ohne Kontakt entspricht, zumindest eines von:
Anpassen von Daten, die durch die tragbare Rechenvorrichtung (100) erzeugt werden und die während des Zustands (408) am Handgelenk ohne Kontakt aufgenommen werden; oder
Anpassen eines Konfidenzwerts für die Daten, die durch die tragbare Rechenvorrichtung (100) erzeugt werden und die während des Zustands (408) am Handgelenk ohne Kontakt aufgenommen werden.

10. Computerimplementiertes Verfahren nach Anspruch 2, wobei Bestimmen, dass der Tragezustand der tragbaren Rechenvorrichtung (100) dem Zustand (404) nicht am Handgelenk entspricht, beinhaltet:
Bestimmen, über den einen oder die mehreren Prozessoren, dass sich die tragbare Rechenvorrichtung (100) nicht mehr in der Nähe der Haut des Benutzers befindet, basierend, zumindest teilweise, auf den zweiten Näherungsdaten;
Bestimmen, über den einen oder die mehreren Prozessoren, dass der Impedanzsensor die Haut des Benutzers nicht kontaktiert, basierend, zumindest teilweise, auf den Impedanzdaten; und
als Reaktion auf Bestimmen, dass sich die tragbare Rechenvorrichtung (100) nicht weiterhin in der Nähe der Haut des Benutzers befindet und der Impedanzsensor die Haut des Benutzers nicht kontaktiert, Bestimmen, über den einen oder die mehreren Prozessoren, dass der Tragezustand für die tragbare Rechenvorrichtung (100) einem Zustand (404) nicht am Handgelenk entspricht.

11. Computerimplementiertes Verfahren nach Anspruch 10, ferner umfassend:
Deaktivieren, über den einen oder die mehreren Prozessoren, des Impedanzsensors als Reaktion auf Bestimmen, dass der Tragezustand für die tragbare Rechenvorrichtung (100) dem Zustand (402) nicht am Handgelenk entspricht und sich nicht mehr in der Nähe der Haut des Benutzers befindet.

12. Tragbare Rechenvorrichtung (100), umfassend:
einen Näherungssensor;
einen Impedanzsensor; und
einen oder mehrere Prozessoren, die konfiguriert sind, um:
erste Näherungsdaten zu erhalten, die durch den Näherungssensor während einer ersten Zeitspanne erzeugt werden;
den Impedanzsensor basierend, zumindest teilweise, auf den ersten Näherungsdaten zu aktivieren;
Impedanzdaten zu erhalten, die durch den Impedanzsensor während einer zweiten Zeitspanne erzeugt werden, die nach der ersten Zeitspanne auftritt;
zweite Näherungsdaten zu erhalten, die durch den Näherungssensor während der zweiten Zeitspanne erzeugt werden; und
zu bestimmen, dass ein Tragezustand der tragbaren Rechenvorrichtung (100) einem von einem Zustand (402, 404) nicht am Handgelenk, einem Zustand (406) am Handgelenk mit Kontakt oder einem Zustand (408) am Handgelenk ohne Kontakt entspricht, basierend, zumindest teilweise, auf den Impedanzdaten und den zweiten Näherungsdaten.

13. Tragbare Rechenvorrichtung (100) nach Anspruch 12, wobei der Näherungssensor einen oder mehrere Ultraschallsensoren, induktive Sensoren oder beides oder einen optischen Sensor beinhaltet.

14. Tragbare Rechenvorrichtung (100) nach Anspruch 12, ferner umfassend:
eine Abdeckung (105; 109); und
ein Tönungselement (107) zwischen der Abdeckung (105; 109) und zumindest dem Näherungssensor,
wobei der eine oder die mehreren Prozessoren ferner konfiguriert sind, das Tönungselement (107) zu steuern, um es basierend zumindest teilweise auf dem Tragezustand zwischen einem transparenten Zustand und einem opaken Zustand wechseln zu lassen.

15. Tragbare Rechenvorrichtung (100) nach Anspruch 12, wobei der Impedanzsensor zumindest eine erste Elektrode (112) und eine zweite Elektrode (112) umfasst, die voneinander beabstandet sind,
wobei die Impedanzdaten anzeigend für einen Kontakt an der ersten Elektrode (112) und einen Kontakt an der zweiten Elektrode (112) sind, optional
wobei sich der Näherungssensor zwischen der ersten und der zweiten Elektrode (112) befindet oder
die erste und die zweite Elektrode (112) aus einem transparenten Material bestehen, wobei die erste und die zweite Elektrode (112) über dem Näherungssensor positioniert sind.

## Revendications

1. Procédé mis en œuvre par ordinateur de détermination d'un état de port d'un dispositif informatique portable (100), le procédé comprenant :
l'obtention, par un ou plusieurs processeurs du dispositif informatique portable (100), des premières données de proximité générées par un capteur de proximité du dispositif informatique portable (100) pendant une première période de temps ;
l'activation, par l'intermédiaire des un ou plusieurs processeurs, d'un capteur d'impédance du dispositif informatique portable (100) sur la base, au moins en partie, des premières données de proximité ;
l'obtention, par l'intermédiaire des un ou plusieurs processeurs, de données d'impédance générées par le capteur d'impédance pendant une seconde période de temps survenant après la première période de temps ;
l'obtention, par l'intermédiaire des un ou plusieurs processeurs, de deuxièmes données de proximité générées par le capteur de proximité pendant la seconde période de temps ; et
la détermination, par l'intermédiaire d'un ou de plusieurs processeurs, du fait que l'état de port du dispositif informatique portable (100) correspond à l'un d'un état hors poignet (402, 404), d'un état au poignet avec contact (406) ou d'un état au poignet sans contact (408), sur la base, au moins en partie, des données d'impédance et des deuxièmes données de proximité.

2. Procédé informatisé selon la revendication 1, dans lequel l'activation du capteur d'impédance sur la base des premières données de proximité comporte :
le fait de déterminer, par l'intermédiaire des un ou plusieurs processeurs, si le dispositif informatique portable (100) est à proximité de la peau d'un utilisateur sur la base, au moins en partie, des premières données de proximité ; et
l'activation, par l'intermédiaire des un ou plusieurs processeurs, du capteur d'impédance en réponse à la détermination du fait que le dispositif informatique portable (100) est à proximité de la peau de l'utilisateur sur la base, au moins en partie, des premières données de proximité.

3. Procédé mis en œuvre par ordinateur selon la revendication 2, dans lequel la détermination du fait que l'état de port du dispositif informatique portable (100) correspond à l'état au poignet avec contact (406) comporte :
la détermination, par l'intermédiaire des un ou plusieurs processeurs, du fait que le dispositif informatique portable (100) est toujours à proximité de la peau de l'utilisateur sur la base, au moins en partie, des deuxièmes données de proximité ; la détermination, par l'intermédiaire des un ou plusieurs processeurs, du fait que le capteur d'impédance est en contact avec la peau de l'utilisateur sur la base, au moins en partie, des données d'impédance ; et
en réponse à la détermination du fait que le dispositif informatique portable (100) est toujours à proximité de la peau de l'utilisateur et que le capteur d'impédance est en contact avec la peau de l'utilisateur, la détermination, par l'intermédiaire des un ou plusieurs processeurs, du fait que l'état de port pour le dispositif informatique portable (100) correspond à l'état au poignet avec contact (406),
éventuellement dans lequel la détermination du fait que le capteur d'impédance est en contact avec la peau de l'utilisateur comporte la détermination, par l'intermédiaire des un ou plusieurs processeurs, du fait que les valeurs d'admittance incluses dans les données d'impédance sont dans une plage de valeurs d'admittance incluses dans les données d'impédance.

4. Procédé mis en œuvre par ordinateur selon la revendication 2, dans lequel la détermination du fait que l'état de port du dispositif informatique portable (100) correspond à l'état au poignet sans contact (408) comporte :
la détermination, par l'intermédiaire des un ou plusieurs processeurs, du fait que le dispositif informatique portable (100) est toujours à proximité de la peau de l'utilisateur sur la base, au moins en partie, des deuxièmes données de proximité ;
la détermination, par l'intermédiaire des un ou plusieurs processeurs, du fait que le capteur d'impédance n'est pas en contact avec la peau de l'utilisateur sur la base, au moins en partie, des données d'impédance ; et
en réponse à la détermination du fait que le dispositif informatique portable (100) est toujours à proximité de la peau de l'utilisateur et que le capteur d'impédance n'est pas en contact avec la peau de l'utilisateur, la détermination, par l'intermédiaire des un ou plusieurs processeurs, du fait que l'état de port pour le dispositif informatique portable (100) correspond à l'état au poignet sans contact (408).

5. Procédé mis en œuvre par ordinateur selon la revendication 4, dans lequel la détermination du fait que le capteur d'impédance n'est pas en contact avec la peau de l'utilisateur comporte la détermination, par l'intermédiaire des un ou plusieurs processeurs, du fait que les valeurs d'admittance incluses dans les données d'impédance sont en dehors d'une plage de valeurs d'admittance indiquant que le capteur d'impédance est en contact avec la peau de l'utilisateur.

6. Procédé mis en œuvre par ordinateur selon la revendication 4, comprenant en outre :
après la détermination du fait que l'état de port du dispositif informatique portable (100) correspond à l'état au poignet sans contact (408), la génération, par l'intermédiaire des un ou plusieurs processeurs, d'une notification pour inviter l'utilisateur à resserrer un bracelet (103) du dispositif informatique portable (100).

7. Procédé mis en œuvre par ordinateur selon la revendication 6, dans lequel une notification invite l'utilisateur à effectuer un geste prédéfini pour confirmer le serrage du bracelet (103).

8. Procédé mis en œuvre par ordinateur selon la revendication 7, comprenant en outre :
l'obtention, par l'intermédiaire des un ou plusieurs processeurs, des données de mouvement générées par un ou plusieurs capteurs de mouvement du dispositif informatique portable, les données de mouvement indiquant que l'utilisateur effectue le geste prédéfini ; et
l'obtention, par l'intermédiaire des un ou plusieurs processeurs, de secondes données d'impédance en réponse à l'obtention des données de mouvement indiquant que l'utilisateur effectue le geste prédéfini, les secondes données d'impédance étant générées par le capteur d'impédance ;
l'obtention, par l'intermédiaire des un ou plusieurs processeurs, des troisièmes données de proximité en réponse à l'obtention des données de mouvement indiquant que l'utilisateur effectue le geste prédéfini, les troisièmes données de proximité étant générées par le capteur de proximité ; et
la re-détermination, par l'intermédiaire des un ou plusieurs processeurs, de l'état de port du dispositif informatique portable sur la base, au moins en partie, des deuxièmes données d'impédance et des troisièmes données de proximité.

9. Procédé mis en œuvre par ordinateur selon la revendication 4, comprenant en outre, après la détermination du fait que l'état de port du dispositif informatique portable (100) correspond à l'état au poignet sans contact (408), au moins l'un :
d'un ajustement des données générées par le dispositif informatique portable (100) enregistrées pendant l'état au poignet sans contact (408) ; ou
d'un ajustement d'une valeur de confiance pour les données générées par le dispositif informatique portable (100) enregistrées pendant l'état au poignet sans contact (408).

10. Procédé mis en œuvre par ordinateur selon la revendication 2, dans lequel la détermination du fait que l'état de port du dispositif informatique portable (100) correspond à l'état hors poignet (404) comporte :
la détermination, par l'intermédiaire des un ou plusieurs processeurs, du fait que le dispositif informatique portable (100) n'est plus à proximité de la peau d'un utilisateur sur la base, au moins en partie, des deuxièmes données de proximité ;
la détermination, par l'intermédiaire des un ou plusieurs processeurs, du fait que le capteur d'impédance n'est pas en contact avec la peau de l'utilisateur sur la base, au moins en partie, des données d'impédance ; et
en réponse à la détermination du fait que le dispositif informatique portable (100) n'est plus à proximité de la peau de l'utilisateur et que le capteur d'impédance n'est pas en contact avec la peau de l'utilisateur, la détermination, par l'intermédiaire des un ou plusieurs processeurs, du fait que l'état de port pour le dispositif informatique portable (100) correspond à un état hors poignet (404).

11. Procédé mis en œuvre par ordinateur selon la revendication 10, comprenant en outre : la désactivation, par l'intermédiaire des un ou plusieurs processeurs, du capteur d'impédance en réponse à la détermination du fait que l'état de port pour le dispositif informatique portable (100) correspond à l'état hors poignet (402) et n'est plus à proximité de la peau de l'utilisateur.

12. Dispositif informatique portable (100), comprenant :
un capteur de proximité ;
un capteur d'impédance ; et
un ou plusieurs processeurs configurés pour :
obtenir les premières données de proximité générées par le capteur de proximité pendant une première période de temps ;
activer le capteur d'impédance sur la base, au moins en partie, des premières données de proximité ;
obtenir des données d'impédance générées par le capteur d'impédance pendant une seconde période de temps survenant après la première période de temps ;
obtenir des deuxièmes données de proximité générées par le capteur de proximité pendant la seconde période de temps ; et
déterminer qu'un état de port du dispositif informatique portable (100) correspond à l'un d'un état hors poignet (402, 404), d'un état au poignet avec contact (406) ou d'un état au poignet sans contact (408), sur la base, au moins en partie, des données d'impédance et des deuxièmes données de proximité.

13. Dispositif informatique portable (100) selon la revendication 12, dans lequel le capteur de proximité comporte un ou plusieurs capteurs à ultrasons, capteurs inductifs, ou les deux, ou un capteur optique.

14. Dispositif informatique portable (100) selon la revendication 12, comprenant en outre :
un couvercle (105 ; 109) ; et
un élément de teinte (107) entre le couvercle (105 ; 109) et au moins le capteur de proximité,
dans lequel les un ou plusieurs processeurs sont en outre configurés pour contrôler l'élément de teinte (107) afin de passer d'un état transparent à un état opaque sur la base au moins en partie de l'état de port.

15. Dispositif informatique portable (100) selon la revendication 12, dans lequel le capteur d'impédance comprend au moins une première électrode (112) et une seconde électrode (112) espacées l'une de l'autre,
dans lequel les données d'impédance indiquent le contact au niveau de la première électrode (112) et le contact au niveau de la seconde électrode (112), éventuellement
dans lequel le capteur de proximité est entre la première et la seconde électrode (112) ou
les première et seconde électrodes (112) sont faites d'un matériau transparent, les première et seconde électrodes (112) étant positionnées au-dessus du capteur de proximité.
